(19) Europäisches Patentamt
European Patent Office
Office européen des brevets

(11) EP 3 760 291 B1

(12) **EUROPEAN PATENT SPECIFICATION**

(45) Date of publication and mention of the grant of the patent:
**30.03.2022 Bulletin 2022/13**

(21) Application number: **20183410.8**

(22) Date of filing: **01.07.2020**

(51) International Patent Classification (IPC):
***B01D 11/02*** *(2006.01)* ***A23L 33/105*** *(2016.01)*
***A61K 36/48*** *(2006.01)* ***A61K 36/73*** *(2006.01)*
***A61K 36/87*** *(2006.01)* ***B01D 9/00*** *(2006.01)*

(52) Cooperative Patent Classification (CPC):
**B01D 11/0288; A23L 33/105; A61K 36/48; A61K 36/73; A61K 36/87; B01D 9/0054; B01D 11/028;** A61K 2236/00; B01D 11/0292

(54) **METHOD FOR SEPARATING RESVERATROL FROM BIOMASS**

VERFAHREN ZUR ABTRENNUNG VON RESVERATROL AUS BIOMASSE

PROCÉDÉ DE SÉPARATION DU RESVÉRATROL DE LA BIOMASSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2019 ZA 201904323**

(43) Date of publication of application:
**06.01.2021 Bulletin 2021/01**

(73) Proprietor: **Stellenbosch University**
**Western Cape Province 7600 (ZA)**

(72) Inventor: **POTT, Robert William McClelland**
**7600 Stellenbosch (ZA)**

(74) Representative: **Schiweck Weinzierl Koch Patentanwälte Partnerschaft mbB**
**Ganghoferstraße 68 B**
**80339 München (DE)**

(56) References cited:
**CN-A- 107 840 839**

- **LUCÍA XAVIER ET AL: "Application of aqueous two phase systems based on polyethylene glycol and sodium citrate for the recovery of phenolic compounds from Eucalyptus wood", MADERAS. CIENCIA Y TECNOLOGÍA, no. ahead, 1 January 2015 (2015-01-01), pages 0-0, XP055749908, DOI: 10.4067/S0718-221X2015005000032**
- **SAILI J. INDURKAR ET AL: "Aqueous two-phase extraction of punicalagin ([alpha]+[beta]) from pomegranate peel by response surface methodology", SEPARATION SCIENCE AND TECHNOLOGY, vol. 54, no. 1, 27 September 2018 (2018-09-27), pages 51-58, XP055750085, US ISSN: 0149-6395, DOI: 10.1080/01496395.2018.1488866**
- **XAVIER LUCÍA ET AL: "Recovery of Phenolic Compounds fromEucalyptus globulusWood Wastes using PEG/phosphate Aqueous Two-Phase Systems", WASTE AND BIOMASS VALORIZATION, SPRINGER NETHERLANDS, NL, vol. 8, no. 2, 24 May 2016 (2016-05-24), pages 443-452, XP036155465, ISSN: 1877-2641, DOI: 10.1007/S12649-016-9579-0 [retrieved on 2016-05-24]**
- **MARIO A. TORRES-ACOSTA ET AL: "Aqueous Two-Phase Systems at Large Scale: Challenges and Opportunities", BIOTECHNOLOGY JOURNAL, vol. 14, no. 1, 1 January 2019 (2019-01-01), page 1800117, XP055750320, DE ISSN: 1860-6768, DOI: 10.1002/biot.201800117**
- **JACQUELINE HERBST ET AL: "The Effect of Temperature on Different Aqueous Two-Phase Diagrams of Polyethylene Glycol (PEG 6000, PEG 8000, and PEG 10000) + Potassium Sodium Tartrate + Water", JOURNAL OF CHEMICAL AND ENGINEERING DATA., vol. 64, no. 7, 18 June 2019 (2019-06-18), pages 3036-3043, XP055750323, US ISSN: 0021-9568, DOI: 10.1021/acs.jced.9b00133**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **HUI WANG ET AL: "Microwave-assisted aqueous two-phase extraction of piceid, resveratrol and emodin from Polygonum cuspidatum by ethanol/ammonium sulphate systems", BIOTECHNOLOGY LETTERS, SPRINGER NETHERLANDS, DORDRECHT, vol. 30, no. 12, 6 August 2008 (2008-08-06), pages 2079-2084, XP019639499, ISSN: 1573-6776, DOI: 10.1007/S10529-008-9815-1**

## Description

### FIELD OF THE INVENTION

This invention relates to a method for separating polyphenolic compounds from biomass, the products resulting from the method and the use of such products in dietary supplements or medicaments.

### BACKGROUND TO THE INVENTION

Polyphenolic compounds (also known as polyphenols or polyhydroxyphenols) are a structural class of mainly natural chemicals characterized by the presence of multiple phenol structural units. The number and characteristics of these phenol structures underlie the unique physical, chemical, and biological properties of particular members of the class. Examples of polyphenolic compounds include resveratrol, tannic acid and ellagitannin. The chemical class of tannins is a subset of the polyphenols.

Resveratrol, in particular, is a hydroxylated stilbene derivative that is naturally present in various plants and food sources such as peanuts, cocoa, berries, soy and grapes. Resveratrol is a phytoalexin, i.e. it is an anti-bacterial and anti-fungal compound produced by plants in response to adverse conditions. This polyphenolic compound has two geometric isomers and can exist in either *trans*- or *cis*- molecular configurations. The *trans*- isomer (also referred to herein as trans-resveratrol or (*E*)-resveratrol) undergoes photoisomerization to the *cis*- isomer (also referred to herein as *cis*-resveratrol or (*Z*)-resveratrol) when exposed to ultraviolet (UV) light.

Due to the molecule being photoisomerizable, the term "resveratrol", as used herein, should be interpreted to encompass one or both of the *trans*- isomeric form (I) and the *cis*- isomeric form (II) having the following chemical structures:

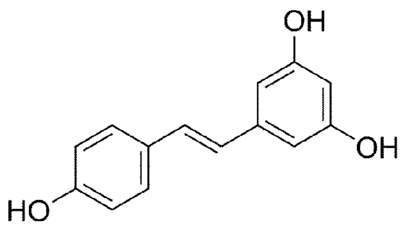

(I) *trans*- isomeric form

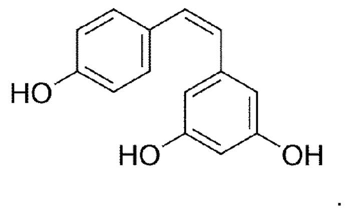

(II) *cis*- isomeric form

Resveratrol has medicinal properties such as anti-oxidant, anti-inflammatory, chemotherapeutic, anticarcinogenic and estrogenic properties. The *trans*- configuration is a stable molecule which the human body can absorb, while the *cis*- configuration of resveratrol is not as easily absorbed (Orallo, 2006). The *trans*- isomer is the predominant form found in grapes and vines (Fremont, 2000). Various preclinical studies have shown that *trans*-resveratrol is biologically active and may find use in the treatment and prevention of cardiovascular disease, liver disease, Alzheimer's disease and various other degenerative diseases (Bosutti and Degens, 2015; Bishayee et al., 2010). Resveratrol appears to also have anti-cancer and anti-aging properties (Matos, 2013). *Trans*-resveratrol has been shown to inhibit the growth of cancer cells in animal models and in cultures (Bishayee, 2009; Aggarwal et al., 2004). Resveratrol also prolongs the lifespan of *Saccharomyces cerevisiae* (Wood et al., 2004) and retard the expression of age-dependent traits in vertebrates (Valenzano et al., 2006).

*Cis*-resveratrol has not been linked to all of the health benefits associated with the *trans*-isomer, but may find use as an antiplatelet agent (Rossi et al., 2012). It has also been reported that methylation of the *cis*- isomer resulted in a derivative with potent anti-cancer properties, highlighting the fact that the *cis* isomer and its derivatives may also find use as a nutraceutical compound (Morris et al., 2015).

Resveratrol is widely used as a dietary supplement and there is a growing demand for the compound in a pure form.

In particular, resveratrol is a valuable chemical constituent of wine grapes. There have been some studies on the resveratrol content of various parts of solid winery waste. These wastes may be a source of both resveratrol, and other phenolic compounds, for use in nutraceutical production (Devesa-Rey et al., 2011; Beres et al., 2017).

However, 90% of the resveratrol from wine grapes is discarded with the waste must after the wine making process. Grape pomace (the grape skins, grape seeds and residual juice that remain after the pressing of grapes), grape canes (shoots or stems) and leaves are all sources of resveratrol. Resveratrol may be extracted from waste biomass produced by the wine industry to provide a valuable co-product. Large amounts of natural wine waste are generated in

the wine-making process when the grapes are crushed, when the vines are pruned or the vines are discarded. It has been determined that 116 800 t/y of wine pomace and 33 700 t/y of grape canes are produced by the wine industry in the Stellenbosch wine region in South Africa. There is an estimated total resveratrol content of 2400 t contained within the biomass.

Grape skins contain significant amounts of resveratrol (Burns et al., 2002; Threlfall et al., 1999). The amount of resveratrol in grape skins depends on a number of factors such as the grape cultivar, geographic location and environmental conditions, as well as exposure to disease and adverse conditions. Red grapes tend to have a higher resveratrol content in comparison to white grapes, with concentrations of the *trans-* isomer ranging between 0.1 and 15 mg/L in red wine (Fremont, 2000). Resveratrol is produced in plants in response to unfavourable environmental conditions and fungal or pathogen infections. According to Romero-Perez et al. (2001), the trans-resveratrol content in grape berries infected with powdery mildew is up to 12-fold higher in severely infected grape berries when compared to healthy grapes. The degree of infection was found to be related to the stilbene content of the grapes. Grapes that would otherwise be considered as waste due to severe infection would present favourable resveratrol yields, highlighting the potential extraction of the biomolecule from viticulture waste.

Significant quantities of *trans*-resveratrol is also present in grape canes (Soural et al., 2015). The canes refer to the shoots or stems of the grapevine that have reached about a year in age and have lost most of their leaves. Canes of white variety grapes in Southern Chile include 7857 $\pm$ 172 mg/kg stilbenes and that this amount can increase if the canes are left for some time on the vineyard after pruning (Vergara et al., 2012). An increase in resveratrol content was observed during the first two months, with a decrease seen after this period (Vergara et al., 2012).

Generally, the lifespan of grapevines is approximately 25 years. The vines are more susceptible to disease with age. Once the plants are infected, it is difficult to restore them back to health and they are often discarded or burnt. Such discarded grapevines may be a useful source of resveratrol.

Resveratrol can be synthesized, obtained by fermentation or extracted from plants.

Current methods of separating resveratrol from wine must involve organic solvent extraction. Water and organic solvents such as ethanol, methanol, ethyl acetate and dichloromethane are commonly used to create solvent extraction systems. A biomass sample is added to a water-solvent mixture to extract the resveratrol therefrom. Sufficient contact time must be allowed for the resveratrol to be extracted from the solid phase into the liquid phase. Further purification and concentration methods are carried out to recover the resveratrol in an isolated form.

Grape canes have been investigated as a source of *trans*-resveratrol (Rayne et al., 2008). Aqueous alcoholic solvent systems such as ethanol and water were used to extract resveratrol from the grape canes. An extraction yield of 3.45 $\pm$ 0.04 mg/g dry weight of *trans*-resveratrol was obtained from *Vitis vinifera* L. cv Pinot Noir grape canes using an ethanol-water solvent system.

In addition to such ethanol-water systems, numerous other solvent extraction methods to extract resveratrol have been investigated. A preparative process investigated by Wang et al. (2013) included reflux extraction, filtering, hydrolyzing under hydrochloric acid conditions, liquid-liquid extraction, eluting with an alkaline aqueous solution of pH 8-9 to remove impurities followed by phase separation of the mixture in a funnel to obtain resveratrol from *P. Cuspidatum.* A recovery of 73.8% resveratrol in the final product was achieved by the extraction recovery (Wang et al., 2013).

Soural et al. (2015) have compared several extraction methods to obtain secondary stilbene derivatives from viticulture waste products. Extraction methods investigated included maceration at laboratory and elevated temperatures, fluidized-bed extraction, Soxhlet extraction as well as microwave-assisted and accelerated solvent extraction. Various conditions were also studied, including the use of different solvents, powdered versus cut cane material, the use of different extraction times and the use of singular versus multiple extractions to obtain trans-resveratrol from grape canes. The extraction was performed using methanol as a protic solvent. The extraction power of methanol was compared with acetone as an aprotic solvent. The highest concentration of trans-resveratrol obtained was 6030 $\pm$ 680 $\mu$g/g dry weight. The highest amount of stilbenes was obtained with accelerated solvent extraction in methanol with powdered source material.

Existing organic solvent extraction methods are cumbersome to implement on an industrial scale due to their cost, complexity and environmental impact. A further challenge encountered with organic solvent extraction methods is the subsequent concentration of the target polyphenolic compound such as resveratrol from the solvent phase. Further process steps are generally required to then formulate the polyphenolic compound into a supplement form. Many natural polyphenolic compounds, including resveratrol, are sensitive to temperature, pH, visible and UV light. Resveratrol, for example, and may degrade and isomerise to the *cis-* configuration under the conditions of organic solvent extraction and subsequent concentration and formulation.

Lucia Xavier et al., Maderas. Ciencia Y Technologia 2015, 0-0 discloses the application of aqueous two phase systems based on polyethylene glycol and sodium citrate for the recovery of phenolic compounds from Eucalyptus wood.

Saili J. Idurkar et al., Separation Science and Technology 2018, vol. 54, no. 1, 51-58 discloses the aqueous two-phase extraction of punicalagin from pomegranate peel by response surface technology.

Xavier Lucia et al., Waste and Biomass Valorization 2016, vol. 8, no. 2, 443-452 discloses recovery of phenolic

compounds from eucalyptus globulus wood wastes using PEG/phosphate aqueous two-phase systems.

CN 107 840 839 A discloses the two-phase aqueous extraction combined with forward osmosis condensation and extraction technology for blueberry leaf anthocyanidin.

Mario A. Torres-Acosta et al., Biotechnology Journal 2019, vol. 14, no. 1, 1800117 discloses aqueous two-phase systems at large scale; challenges and opportunities.

Jacqueline Herbst et al., Journal of Chemical Engineering Data. 2019, vol. 64, no. 7 discloses the effect of temperature on different aqueous two-phase diagrams of polyethylene glycol + potassium sodium tartrate + water.

Hui Wang et al. Biotechnology letters, 2008, vol.30, no. 12, 2079-2084 discloses microwave-assisted aqueous two-phase extraction of piceid, resveratrol and emodin from Polygonum cuspidatum by ethanol/ammonium sulphate systems.

The preceding discussion of the background to the invention is intended only to facilitate an understanding of the present invention. It should be appreciated that the discussion is not an acknowledgment or admission that any of the material referred to was part of the common general knowledge in the art as at the priority date of the application.

## SUMMARY OF THE INVENTION

In accordance with the invention there is provided a method for separating a polyphenolic compound from biomass, the method comprising feeding the biomass into an aqueous two-phase system with a polymer phase and a salt phase and separating the polymer phase which includes the polyphenolic compound from the salt phase, wherein the polyphenolic compound is resveratrol and the salt is selected from the group consisting of a tartrate salt, a citrate salt, a carbonate salt, a phosphate salt, a sulphate salt, and any combinations thereof.

The polymer may be a polyether. The polymer may be selected from the group consisting of polyethylene glycol (PEG), polypropylene glycol (PPG), and combinations thereof.

The salt may be selected from the group consisting of potassium sodium tartrate, sodium citrate, potassium phosphate, ammonium sulphate, and any combinations thereof.

The biomass may be selected based on the content of a target polyphenolic compound therein. The target polyphenolic compound is resveratrol. The biomass may be derived from at least one plant selected from the group consisting of grape vines, knotweeds, pine trees, peanut plants, cocoa bushes, *Vaccinium* shrubs that produce berries, geraniums of the *Pelargonium* genus, the herb *Polygonum Cuspidatum,* and any combinations thereof. The biomass may be selected from the group consisting of grape pomace, grape canes, vine leaves, peanuts, cocoa powder, blueberries, raspberries, mulberries, cranberries, bilberries, geraniums of the *Pelargonium* genus, the herb *Polygonum Cuspidatum,* and any combinations thereof. The biomass may comprise winery waste.

The biomass may be milled prior to feeding it into the aqueous two-phase system. The biomass may be homogenised prior to feeding it into the aqueous two-phase system.

The aqueous two-phase system may have relative proportions selected to enhance resveratrol separation from the biomass.

The polymer phase may comprise from about 1% to about 80% (w/w) polyethylene glycol, preferably from about 5% to about 15% (w/w) polyethylene glycol. The polymer phase may comprise about 10% (w/w) polyethylene glycol.

The polyethylene glycol may have a molecular weight in a range from about 400 g/mol to about 20,000 g/mol, preferably in a range from about 400 g/mol to about 10,000 g/mol. Preferably still, the polyethylene glycol may have a molecular weight in a range from about 4000 g/mol to about 10 000 g/mol. The polyethylene glycol may have a molecular weight of about 8000 g/mol.

The salt phase may comprise from about 5% to about 50% (w/w) tartrate salt, preferably from about 10% to about 20% (w/w) tartrate salt. The salt phase may comprise about 15% (w/w) tartrate salt.

The method may include a step of recovering the polyphenolic compound at least partially from the polymer phase.

The step of recovering the polyphenolic compound from the polymer phase may be performed by protein precipitation to yield a polyphenol-protein precipitate. The protein precipitation may be performed with a protein source selected from the group consisting of albumin, tryptone soy broth, yeast extract, and any combinations thereof.

The method may include a further step of drying the polyphenol-protein precipitate.

The step of recovering the polyphenolic compound from the polymer phase may, instead or in addition, involve feeding the polymer phase into a second aqueous two-phase system with a second polymer phase and separating the second polymer phase which includes the polyphenolic compound from the polymer phase fed into the second aqueous two-phase system. The second polymer phase may comprise a carbohydrate. The second polymer phase may comprise a carbohydrate selected from the group consisting of maltodextrin and dextran.

The method may include a further step of drying the second polymer phase which includes the polyphenolic compound to yield a polyphenol-polymer powder.

The method may include recycling the polymer phase after at least partially recovering the polyphenolic compound therefrom, and reusing the polymer phase in the aqueous two-phase system to separate a polyphenolic compound from the biomass.

The method may include recycling the salt phase of the aqueous two-phase system. The method may include separating the biomass from the salt phase and reusing the salt phase in the aqueous two-phase system to separate a polyphenolic compound from the biomass.

It is further disclosed herein a polyphenol-protein precipitate obtained by protein precipitation according to the method described above

It is further disclosed herein a medicament which includes the polyphenol-protein precipitate. It is further disclosed herein a dietary supplement which includes the polyphenol-protein precipitate.

It is further disclosed herein a polyphenol-polymer powder obtained from the second aqueous two-phase system according to the method described above, said polyphenol-polymer powder including the second polymer.

It is further disclosed herein a medicament which includes the polyphenol-polymer powder.

It is further disclosed herein a dietary supplement which includes the polyphenol-polymer powder.

Modes for performing the method of the invention will now be described, by way of example only, with reference to the accompanying drawings.

## BRIEF DESCRIPTION OF THE DRAWINGS

In the drawings:

Figure 1 is a schematic flow diagram illustrating an exemplary mode of performing a method for separating resveratrol from biomass with a protein precipitation recovery step;

Figure 2 is a schematic flow diagram that illustrates an exemplary mode of performing a method for separating resveratrol from biomass with a recovery step that involves a polymer-polymer aqueous two-phase system;

Figure 3 is an illustration of a binodal curve of a polymer-salt ATPS;

Figure 4 is a bar graph of the concentration of resveratrol in various winery waste samples;

Figure 5 is a bar graph of the resveratrol partition coefficient for a range of different PEG molecular weights in PEG-ammonium sulphate ATP systems with system A containing 11 wt% PEG and 26 wt% ammonium sulphate and system B containing 13 wt% PEG and 13 wt% ammonium sulphate;

Figure 6 is a bar graph of the resveratrol extraction achieved for a range of different PEG molecular weights in PEG-ammonium sulphate ATP systems with system A containing 11 wt% PEG and 26 wt% ammonium sulphate and system B containing 13 wt% PEG and 13 wt% ammonium sulphate;

Figure 7 is a plot of the resveratrol partition coefficient to the PEG 8000 top phase for different ammonium sulphate salt concentrations;

Figure 8 is a plot of the percentage resveratrol extraction achieved to the PEG 8000 top phase for different ammonium sulphate salt concentrations;

Figure 9 is a bar graph of the resveratrol partition coefficient to the 11 wt% PEG top phase with 26 wt% tartrate, citrate, sulphate and phosphate salt solutions;

Figure 10 is a bar graph of the percentage resveratrol extracted to the 11 wt% PEG top phase with 26 wt% tartrate, citrate, sulphate and phosphate salt solutions;

Figure 11 is a plot of the resveratrol recovery concentration over a range of tryptone soy broth and yeast extract concentrations;

Figure 12 is a bar graph of the percentage resveratrol recovered from a 1 g/l solution in a 33 wt% PEG / 33 wt% maltodextrin system with 10 g/l of albumin, yeast extract and tryptone soybroth, respectively;

Figure 13 is a plot of the yield of resveratrol separated into the maltodextrin rich phase of a 10% (w/w) PEG 8000 / 35% (w/w) mdx polymer-polymer ATPS and a 10% (w/w) PEG 4000 / 40% (w/w) mdx polymer-polymer

ATPS at different pH values;

Figure 14 is a plot of the concentration of the *trans*- and *cis*- forms of resveratrol in the top PEG 800 phase of a 10% (w/w) PEG 8000 / 35% (w/w) mdx polymer-polymer ATPS at different pH values; and

Figure 15 is a plot of the respective concentration of the *trans*- and *cis*- forms of resveratrol in the bottom mdx-rich phase of a 10% (w/w) PEG 8000 / 35% (w/w) mdx polymer-polymer ATPS at different pH values.

## DETAILED DESCRIPTION WITH REFERENCE TO THE DRAWINGS

An aqueous two-phase system (ATPS), otherwise known as an aqueous two phase extraction (ATPE) or an aqueous biphasic system (ABS) is a liquid-liquid binary water-based extraction system that is formed by the combination of two structurally different, water-soluble polymers, such a polyethylene glycol (PEG) and dextran, or a single polymer and a specific salt such as PEG and potassium sodium tartrate salt. Phase-forming polymers or salts are solubilized in water at suitably high concentrations that are above the critical concentration to form two separate liquid phases. A clear interfacial boundary separates two distinct, immiscible aqueous-based phases, with each phase preferentially rich with one of the polymers or salt. A polymer-polymer ATPS is formed with two immiscible solutions of different polymers, whilst a polymer-salt ATPS is formed with a polymer solution that is incompatible or immiscible with a salt solution. Molecules tend to preferentially partition into one of the phases based on a number of factors such as the surface properties of the molecule, the phase compositions and concentrations, pH and temperature, thereby enabling the purification of a target molecule from mixed solutions.

A method involving ATPS is provided to separate or extract a polyphenolic compound from biomass. Depending on the type and composition of the biomass, a mixture of polyphenolic compounds may be separated from the biomass by carrying out the methods described herein.

In the described method, the biomass includes resveratrol and the resveratrol may be separated from the biomass together with other polyphenolic compounds present in the biomass feed. It will be appreciated that the method of extraction described herein is not limited to the extraction of resveratrol but may be applied for the extraction of numerous other polyphenolic compounds from biomass. In winery waste, for example, other such phenolics may include gallic acid, epicatechin, catechin, quercetin, myricetin, and ellagic acid. These phenolic compounds may be present in different concentrations and ratios in the different parts of a grape vine (Xia et al., 2013).

The following is an example of a methodology for determining the total phenolic content of different biomass samples. Sample of biomass were diluted to make up a volume of 100 μL. Each 100 μL sample was mixed with 200 μL of 10 v/v% Folin-Ciocalteu (FC) reagent for 30 seconds using a vortex mixer. After 30 seconds of mixing, 800 μL of 0.7 M $Na_2CO_3$ solution was added and vortexed again. The samples were stored for 2 hours at ambient temperature. Thereafter, 200 μL of each sample was transferred to a 96-well microplate to be analysed using a BioTek Elx800 spectrophotometer. The absorbance of each sample was converted to concentration in terms of gallic acid equivalent (GAE) with a gallic acid standard curve.

Using an FC assay, the inventor conducted a study of the total phenolic content of Pinotage grape skins, seeds, stems, canes and leaves obtained from the grape harvests of two consecutive years. The total phenolic content was assessed in terms of gallic acid equivalents. For samples from the first year's harvest it was found that the leaves had the highest overall total phenolic content with a maximum of 5.02 ± 0.16 mg/g. The phenolic content in the post-fermentation skins ranged from 3.98 ± 0.081 mg/g to 4.22 ± 0.10 mg/g and from 1.07 ± 0.013 mg/g to 1.4 ± 0.11 mg/g in the cane trimmings. The canes had the highest resveratrol concentration but the lowest total phenolic content, while the skins, leaves and seeds had the lowest resveratrol concentration. For the following year's harvest, the total phenolic content of the stems, leaves, post-fermentation skins and seeds were within a range similar to that of the first year's harvest, while the canes contained up to 2.36 ± 0.22 mg phenolics per gram of dried canes. Total phenolic concentration remained between 4 mg to 5 mg gallic acid per gram of dried skins, seeds, stems and leaves. However, the average phenolic concentration in the cane trimmings increased from 1.22 ± 0.06 mg/g for the first year to 1.95 ± 0.22 mg/g for the subsequent year. By comparing the phenolic and resveratrol concentration it was concluded that there was no correlation between the total phenolic content and resveratrol.

The present method for extracting polyphenolic compounds may comprise feeding the biomass into an ATPS with a polymer phase and a salt phase and allowing the polyphenolic compound to be extracted from the biomass and diffuse into and accumulate in the polymer phase over a select period of time. The contact time of the biomass with the ATPS may be 0.5 hours or more, preferably between 0.5 and 3 hours and most preferably 2 hours. The total contact time may be divided into a mixing period during which the ATPS and biomass is mixed, followed by a settling period during which the biomass is permitted to settle into the salt phase and the extracted polyphenolic compound diffuses into the polymer phase. The method may involve numerous alternating mixing and settling periods within the total contact time period.

After sufficient contact time, the polymer phase which includes one or more polyphenolic compounds may be separated from the salt phase. The polymer phase may be separated from the salt phase by any suitable method such as settling and decanting. In this manner, polymer-salt ATPS may be used to extract polyphenolic compounds from the biomass raw product and separate them from the biomass. The extraction or separation of resveratrol from biomass may be followed by further method steps for recovering and/or isolating the one or more polyphenolic compounds and providing it in a useful form.

The solid biomass may preferably be pre-treated in a suitable manner such as by being milled and/or homogenised prior to feeding it into the aqueous two-phase system to facilitate extraction of a polyphenolic compound therefrom.

The type of biomass may be selected based on it containing a target polyphenolic compound that may be separated from the biomass with a suitable polymer-salt ATPS. The biomass may, for example, be any plant or plant material that contains resveratrol as the target polyphenolic compound. Should at least one of the target polyphenolic compounds be resveratrol, the biomass may be derived from a plant selected from the group consisting of grape vines, knotweeds, pine trees, peanut plants, cocoa bushes, *Vaccinium* shrubs that produce berries, geraniums of the *Pelargonium* genus, the herb *Polygonum Cuspidatum,* and any combinations thereof. A mixture of biomass from different plants may be used. In particular, the method may be carried out with biomass known to have significant quantities of resveratrol per gram dry weight such as a biomass selected from the group consisting of grapes, grape pomace, grape canes, vine leaves, peanuts, cocoa powder, blueberries, raspberries, mulberries, cranberries, bilberries, geraniums of the *Pelargonium* genus, the herb *Polygonum Cuspidatum,* and any combinations thereof. In one mode of performing the described method, the biomass may be winery waste which may be selected from the group consisting of grape pomace, grape canes, vine leaves, and any combinations thereof.

The polymer in the polymer-salt ATPS may be a polyether such as polyethylene glycol (PEG) or polypropylene glycol (PPG). PEG is generally preferred due to its low cost and desirable extraction properties.

The phases of a PEG-salt system may have a significant difference in density, low viscosity and provide greater selectivity to improve the separation.

The PEG may have a molecular weight that ranges between about 400 g/mol and about 20 000 g/mol, preferably between about 400 g/mol and about 10,000 g/mol. Preferably still, the PEG may have a molecular weight that ranges between about 4000 g/mol and 10 000 g/mol. In certain embodiments, PEG with a molecular weight of about 8000 g/mol (PEG 8000) may be used to form the polymer phase.

The salt is selected from the group consisting of a tartrate salt, a citrate salt, a carbonate salt, a phosphate salt, a sulphate salt, and any combinations thereof. In particular, the salt may be potassium sodium tartrate tetrahydrate, a sodium citrate such as trisodium citrate dihydrate, potassium phosphate or ammonium sulphate, preferably a tartrate salt such as potassium sodium tartrate tetrahydrate.

The ratio of polymer to salt may range between the respective minimum and maximum polymer and salt concentrations required to form two separate phases at ambient conditions. These minimum and maximum concentrations may be obtained from the phase diagram of a specific ATPS. For example, with the preferred PEG 8000 polymer phase, the minimum and maximum PEG 8000 and salt concentrations required to form two phases at 298.15 K may be calculated from phase diagrams for various different aqueous two-phase systems as per Table 1.

Table 1. The minimum and maximum PEG 8000 and salt concentrations required to form two phases at 298.15 K.

| ATPS | PEG mass fraction | Salt mass fraction |
|---|---|---|
| PEG 8000 + Potassium sodium tartrate tetrahydrate | 0.24 | 0.09 |
| | 0.03 | 0.22 |
| | 0.53 | 0.02 |
| PEG 8000 + Tri-sodium citrate dihydrate | 0.01 | 0.22 |
| | 0.22 | 0.08 |
| PEG 8000 + Potassium phosphate | 0.14 | 0.12 |
| | 0.54 | 0.01 |
| PEG 8000 + Ammonium sulphate | <0.00 | 0.33 |

The upper limit for the concentration of the salt phase is its saturation in the aqueous solution thereof.

The biomass may be fed into the polymer-salt ATPS and after a sufficient amount of time (which may involve mixing and settling), substantially all of the biomass may migrate to and accumulate in the salt phase, whilst the solubilised polyphenolic compound(s) extracted from the biomass may diffuse to and accumulate in the polymer phase. Polyphenolic

compounds, including resveratrol, may preferentially and selectively partition to the polymer phase due to its hydrophobic nature.

The polymer phase is the top phase, and the salt phase is the bottom phase in a polymer-salt ATPS.

After the polyphenolic compound has been extracted from the biomass and diffused to the polymer phase of the polymer-salt ATPS, the polymer phase may be separated from the salt phase so that the polyphenolic compound may be recovered from the polymer phase. Following recovery of some or all of the polyphenolic compound, the polymer phase may be recycled and reused in the polymer-salt ATPS. Similarly the salt phase with the biomass may be recycled by first separating the biomass from the salt phase by an appropriate separation process such as filtration or centrifugation and then diverting the recycled salt phase back to the aqueous two-phase system to be reused in the extraction of a polyphenolic compound from the biomass. Some of the used polymer or salt phase may be purged as may be required for continual operation of the separation process.

The polyphenolic compound in the polymer phase that has been separated from the salt phase of the first polymer-salt ATPS may be recovered by subsequent protein precipitation or polymer-polymer ATPS. A protein solution may be added to the polymer phase containing the polyphenolic compound to result in the precipitation thereof from the polymer phase in the form a polyphenol -protein precipitate. The protein solution may be a solution of albumin, a tryptone, soy broth, yeast extract, or any combinations thereof. The polyphenol-protein precipitate may be separated from aqueous solution by any suitable separation technique such as filtration or centrifugation. Thereafter, the polyphenol-protein precipitate may be dried, for example, by spray drying or drum drying.

The polyphenol-protein precipitate obtained in terms of the methods described herein may be directly used in the manufacture of a medicament or a health or dietary supplement. The medicament or dietary supplement contains resveratrol, preferably contains mostly resveratrol. A medicament may include a further natural or synthetic medicinal active ingredient, whereas a dietary supplement may include other supplement ingredients such as other biologically beneficial pigments, other polyphenols, probiotics or nutrients such as vitamins, minerals, fibre, fatty acids and amino acids. The medicament or dietary supplement may include an excipient selected from the group consisting of a diluent, a binder, a disintegrant, a glidant, a lubricant, a preservative, a colouring agent, a coating, a film, and any combinations thereof. The medicament or health supplement may be in the form of a pill, capsule or tablet.

As an alternative to protein precipitation, the polyphenolic compound in the polymer phase of the first polymer-salt ATPS may be recovered, or at least partially recovered, by a further separation of the polyphenolic compound by means of polymer-polymer ATPS with a second, recovery polymer phase. The polymer phase separated from the first polymer-salt ATPS may be combined with a solution of a second polymer providing a second polymer phase. In other words, the polymer phase containing the polyphenolic compound extracted from the biomass may be fed into a second aqueous two-phase system. Separation of the polyphenolic compound may be achieved by allowing the polyphenolic compound to diffuse to and preferentially accumulate in the second polymer phase having a greater affinity for the polyphenolic compound than the first or original polymer phase over a select period of time. The total contact time afforded for the second, polymer-polymer ATPS may be 0.5 hours or more, preferably between 0.5 and 3 hours and most preferably less than or 2 hours. The total contact time may be divided into a mixing period of a select duration during which the polymer phases are mixed, followed by a settling period during which the polyphenolic compound is permitted to diffuse to and accumulate in the second polymer phase. Thereafter, the second polymer phase which includes the polyphenolic compound and is richer in the polyphenolic compound than the original polymer phase from the first polymer-salt ATPS may be separated from said original polymer phase fed into the second polymer-polymer ATPS.

The second polymer solution providing the second polymer phase may be dextran or a digestible polymer that is safe for consumption such as a carbohydrate, preferably a dextrin, and more preferably maltodextrin. Maltodextrin is a polysaccharide that is produced by the partial hydrolysis of starch. It is commonly used as a food additive, is easily digestible and water-soluble.

The second polymer phase that is rich in resveratrol following its diffusion and accumulation therein may be separated from the first polymer phase by any suitable method such as draining or decanting. Following this separation, the second polymer phase with accumulated polyphenolic compound may be dried by, for example spray drying or drum drying, to yield a polyphenol-polymer powder.

Depending on the polymers used to form the polymer phases, the second polymer phase will either be the bottom phase or the top phase in the polymer-polymer ATPS. In a dextrin/maltodextrin-PEG ATPS, the dextrin or maltodextrin phase will be at the bottom and the PEG phase will be at the top.

The polyphenol-polymer powder that includes a polymer that is safe for consumption, such as maltodextrin, may be used directly in the manufacture of a medicament or health or dietary supplement as described above with reference to the polymer-protein precipitate. Again, the supplement preferably contains resveratrol and more preferably contains mostly resveratrol amongst other polyphenols. In this regard, both the protein precipitation and the second polymer-carbohydrate ATPS recovery processes result in a polyphenol-matrix and a protein or carbohydrate product, respectively. The polyphenol-matrix containing either a protein or a carbohydrate may be safely consumed without

requiring further refining or recovery steps. The polyphenol-matrix with either a protein or carbohydrate matrix may be directly used as a health supplement. If required, the recovered resveratrol can be isolated or purified to a select degree of purity before its formulation into a supplement or medicament form.

Figure 1 is a schematic diagram of an exemplary method for separating the polyphenolic compound, resveratrol, from biomass with a protein precipitation recovery step. At a first step (101), the biomass containing resveratrol may be milled and/or homogenised to obtain a more uniform biomass feed from which resveratrol can readily be extracted. Homogenization can be accomplished by use of extruders, hammer mills, colloid mills, grinding mills or high shear blenders. At a next step (103), the homogenised biomass may be fed into a polymer-salt ATPS to separate the resveratrol from the biomass. The resveratrol may diffuse into and preferentially accumulate in the polymer phase whilst the biomass migrates into or settles in the salt phase over time. The resveratrol-rich polymer phase may be separated from the polymer-salt ATPS. At a next step (105), a protein solution may be added to the polymer phase which includes resveratrol to precipitate the resveratrol from the polymer phase by forming a resveratrol-protein precipitate. Thereafter, at a further step (107), the resveratrol-protein precipitate may be separated from the aqueous polymer/protein solution and dried to remove any residual water that may be present in the precipitate, to yield a substantially dry resveratrol-protein precipitate that may be directly formulated into a health supplement or the like.

Following the precipitation (105) of the resveratrol with protein, the polymer from the polymer phase may be recycled and reintroduced into the polymer-salt ATPS at a further step (109) to recover any residual resveratrol remaining therein and reuse the polymer. The polymer from the polymer phase may also be purged as may be necessary (111) to prevent build-up. Similarly, the salt from the salt phase may be recycled by first filtering the biomass from the salt phase (113) and then reintroducing the salt back into the polymer salt ATPS. Some of the salt may be purged (115) as may be necessary to avoid build-up.

Figure 2 is a schematic diagram of an exemplary method for separating resveratrol from biomass with a recovery step that involves a polymer-polymer aqueous two-phase system. At a first step (201), the biomass containing resveratrol may be milled and/or homogenised, as was done according to the method described with reference to Figure 1, to obtain a more uniform biomass feed from which resveratrol can readily be extracted. At a next step (203), the milled/homogenised biomass may be fed into a polymer-salt ATPS to separate the resveratrol from the biomass. The resveratrol may diffuse into and selectively accumulate in the polymer phase whilst the biomass migrates to the salt phase over time. The resveratrol-rich polymer phase may be separated from the polymer-salt ATPS and combined with a second polymer solution at further step (205) involving polymer-polymer ATPS. The resveratrol may preferentially diffuse into or partition to the second polymer phase due to favourable intermolecular interactions therewith. The second polymer phase may be separated from the polymer-polymer ATPS by decanting or the like. Thereafter, at a further step (207), the resveratrol-polymer solution may be dried to yield a substantially dry resveratrol-polymer powder, which depending on the type of polymer used, may be directly formulated into a health supplement.

Following the removal of second polymer phase from the polymer-polymer ATPS, the original polymer phase may be recycled (209) and reintroduced into the polymer-salt ATPS to recover some of the resveratrol remaining in the polymer phase in the next cycle. The polymer from the polymer phase may also be purged as may be necessary (211). Similarly, the salt from the salt phase of the polymer-salt ATPS may be recycled by first filtering the biomass from the salt phase (213) and then reintroducing the salt phase back into the polymer salt ATPS. Some of the salt phase or salt may be purged (215) as may be necessary.

The biomass used may be in the form of raw wine waste from wine farms. Such waste is in the form of bulky, non-homogenous crushed grape pomace and stems of grape canes pruned directly from the grape vines. Such biomass samples typically require size reduction to ease processing and maximize the amount of resveratrol that can be extracted. A homogeniser or rotary blender can be used for size reduction and to form a homogenous mixture of grape pomace for extraction. A mill or high shear blender may be required to break the solid materials of grape canes into smaller and more uniform pieces. The uniform biomass may then be transferred to the aqueous two-phase system for resveratrol extraction.

The methods described herein may be carried out by a suitably designed processing plant, such as a polymer-salt ATPS followed by a polymer-polymer ATPS resveratrol recovery plant designed to extract resveratrol, amongst other polyphenolic compounds, from winery waste. The reactors of the plant may have a residence time of 2 hours to allow for 1 hour of mixing and 1 hour of settling. Two reactors may be placed in parallel for the polymer-salt ATPS and the polymer-polymer ATPS to facilitate continuous flow. The total mass flow entering the system may be divided evenly between the two reactors for each of the two ATP systems.

In one embodiment of such a plant, the polymer-salt ATPS may comprise about 10% w/w PEG and about 15% w/w tartrate salt. The polymer-polymer combination that results from addition of the first polymer phase to the second ATPS may comprise about 10% w/w PEG and about 35% w/w maltodextrin (mdx), said w/w ratios being calculated with reference to the water weight of the second two-phase system after addition of the first polymer phase.

In other embodiments, the ratios of PEG to salt may be different from the preferred ratio described above. For example, the polymer phase may comprise from about 1% to about 80% (w/w) PEG, preferably from about 5% to about

15% (w/w) PEG, while the salt phase may comprise from about 5% to about 50% (w/w) tartrate salt, preferably from about 10% to about 20% (w/w) tartrate salt.

The ratio of the polymers in the polymer-polymer combination of the second ATPS may also differ from the preferred ratio stated above. For example, the PEG may be present in an amount from about 1% to about 80% (w/w) while the maltodextrin may be present in an amount from about 10% to about 60% (w/w), said w/w ratios being calculated with reference to the water weight of the second two-phase system after addition of the first polymer phase. Preferably for better recovery, the polymers of the polymer-polymer combination may comprise from about 1% to about 15% (w/w) PEG and from about 10% to about 40% maltodextrin, with the most preferred quantities being about 10% w/w PEG and about 35% w/w maltodextrin as stated above.

Mixer-settlers or column contactors may be utilized as the reactors or process vessels in which the polymer-salt and polymer-polymer ATP processes occur to achieve the desired dispersion, equilibration and phase separation for extraction of resveratrol from the wine waste biomass. PEG, tartrate and water are added to the polymer-salt ATPS. The bottom phase rich in salt solution and remaining biomass exits the reactor and passes over a filter where the biomass and salt solution are separated. The biomass exits the process and a portion of the salt solution is recycled back to the polymer-salt ATPS. The waste biomass may be introduced back into the soil as mulch or utilized for other agricultural processes.

The top-phase of the polymer-salt ATPS rich in resveratrol, amongst the other polyphenols, and PEG may be transferred to the polymer-polymer ATPS reactor, where mdx and additional water are added. A portion of the resultant top phase with PEG and residual resveratrol and other polyphenols may be recycled back to the polymer-salt ATPS to reuse the PEG and further extract the remaining resveratrol and other polyphenols. The bottom phase rich in resveratrol and other polyphenols, and mdx enters a spray drier or drum drier to remove water and produce a dry powder product from the liquid slurry by rapidly drying the mixture.

Instead or in addition, as a second process and plant design option the polymer-polymer ATPS may be substituted with a protein precipitation mechanism to recover resveratrol and other polyphenols from the top phase of the polymer-salt ATPS. The process vessels remain the same, with the protein precipitate containing resveratrol and other polyphenols exiting the bottom of the vessel and following the same process of subsequent purification.

To determine the percentage yield of resveratrol, specifically, from the embodiment of the resveratrol recovery plant described above, the amount of resveratrol in the raw biomass must be determined first. For example, it was determined that grape canes contain 21 mg resveratrol per gram dry weight and that grape pomace includes 14.5 mg resveratrol per gram dry weight. It was found that, with a polymer-salt ATPS comprising about 10% w/w PEG (with a molecular weight of about 8000 g/mol) and about 15% (w/w) tartrate salt, approximately 70% of resveratrol in the winery waste biomass could be extracted and separated from the biomass. The remainder of the resveratrol entering the system may exit in the salt-rich bottom phase contained within the biomass.

It was further determined that with the polymer-polymer ATPS comprising about 10% w/w PEG and about 35% w/w maltodextrin (mdx), a maximum yield of 58% resveratrol into the mdx was obtained. The remaining 42% of the resveratrol in the vessel may be recovered into the top phase of the polymer-salt ATPS by recycling.

The recoveries obtained from ATPS techniques were compared with those obtained from protein precipitations and it was determined that at sufficiently high protein concentrations almost 100% resveratrol recoveries from the polymer phase were obtained.

Recovery of resveratrol can be achieved by protein precipitation with one or more of albumin, tryptone, soy broth, yeast extract, or any combinations thereof. Instead or in addition, recovery can be accomplished with polymer-polymer ATPS with, for example, polyethylene glycol (PEG) and maltodextrin (mdx). The second ATPS with these two polymer phases enables the recovery of resveratrol from the PEG phase into the mdx phase. The concentration of resveratrol into the mdx phase may provide a means of recovery of resveratrol into a supplement form safe for human consumption. The PEG/mdx combination may be a suitable biphasic system for the large-scale processing of resveratrol into a supplement product. The pH and salt content of the system may have a significant influence on the partitioning behaviour and could potentially be manipulated to achieve the desired partitioning and to try and achieve a desired degree of purity in the recovered resveratrol-mdx product.

With both of the different recovery techniques, the PEG phase can be recycled back to the initial PEG-salt ATPS. For example, about 90% of the PEG phase can be recycled back to the first polymer-salt ATPS with a 10% purge stream to prevent build-up. It is desirable to recycle the PEG due to its cost and to recover any remaining resveratrol in the PEG phase. Recycling of PEG may be carried out to save even if the recovery of resveratrol by protein precipitation results in a negligible amount of resveratrol being present in the PEG-recycle stream.

As will be understood by those skilled in the art, suitable polymer-salt ATPS and polymer-polymer ATPS systems may need to be prepared to extract and recover polyphenolic compounds such as resveratrol from biomass. Reliable data on the properties and composition of ATP systems are necessary to design effective extraction processes. Phase diagrams demarcate the potential working area for two-phase systems and are often considered to be a "fingerprint" unique to that system under set conditions. A binodal curve, which is present on the diagram, divides a region of

component concentrations that will form two immiscible aqueous phases (located above the curve) from those that will form one phase (located at, and below the curve). The binodal curve therefore represents the border line between the homogeneous and heterogeneous phases of a ternary mixture. The binodal curve can be determined by the cloud point method, where a concentrated stock solution of salt or polymer (eg. dextran or tartrate) is taken and titrated against a concentrated stock of the other polymer (eg. PEG). At a critical point the mixture becomes turbid or 'cloudy', which is indicative of two-phase formation. Water is then added until the disappearance of turbidity and the procedure is repeated to get the other binodal points.

The liquid-liquid equilibrium gives an indication of the phase forming compositions, which is crucial for the development and scale-up of ATPS extraction processes. There are a number of factors that can be used to modify the phase envelope. It has been observed that biphasic behaviour is highly sensitive to variations in system parameters such as pH, temperature and system composition.

The binodal curve may be used to predict the theoretical composition and volume of both phases. Figure 3 is an illustration of a binodal curve of a PEG-salt system. The binodal curve represents the data required to design ATPS extraction and to predict the partitioning. Point M in Figure 3 represents the mixture point, whereas the points labelled T and B represent the top and bottom phases. The area on the left of the curve represent the polymer (PEG) and salt concentrations (w/w) at which only one phase exists and the area on the right of the curve represent the polymer (PEG) and salt concentrations (w/w) at which two phases will form. At the critical point (C) the volume and composition of both phases will be the same.

The tie line length (TLL) is associated with the mass of the phases. The tie line length can be calculated with equation 1:

$$\mathrm{TLL} = \sqrt{\left[\mathrm{B_{salt}} - \mathrm{T_{salt}}\right]^2 + \left[\mathrm{T_{PEG}} - \mathrm{B_{PEG}}\right]^2} \qquad [1]$$

The slope of the tie line can be calculated with equation 2 in which X is the salt weight fraction and *Y* the polymer weight fraction:

$$\mathrm{STL} = \frac{\mathrm{Y_T} - \mathrm{Y_B}}{\mathrm{X_T} - \mathrm{X_B}} \qquad [2]$$

Equations 3 and 4 may be used to determine the polymer and salt weight percentage when constructing a binodal curve for a system. In equations 3 and 4, x and *y* are the initial mass fractions of the salt and polymer respectively and $m_w$ is the mass of diluent added to the mixture.

$$Y(wt\%) = \frac{y \times m_Y}{m_Y + \Sigma m_X + \Sigma m_w} \times 100\% \qquad [3]$$

$$X(wt\%) = \frac{y \times m_Y}{m_Y + \Sigma m_X + \Sigma m_w} \times 100\% \qquad [4]$$

The partitioning of the polyphenolic biomolecule can be quantified using parameters such as the partition coefficient (K) and the yield (*Y*). The partition coefficient reflects the extent of separation of the polyphenolic compound into the polymer phase. It is defined as the ratio of the equilibrium concentration of the target component in the bottom phase to the equilibrium concentration of the target component in the top phase. Partition coefficients of more than 1 indicate a higher concentration of the specific molecule in the top phase. The partition coefficient (*K*) is calculated with equation 5 in which $c_T$ is the concentration of the molecule in the top phase and $C_B$ is the concentration of the molecule in the bottom phase.

$$K = \frac{C_T}{C_B} \qquad [5]$$

The partitioning of a molecule may depend on factors such as the polymer molecular weight, the concentration of the polymer, the salt ionic strength, the pH and the addition of further salts.

The percentage yield can be used to determine the efficiency of the ATPS in extracting the target biomolecule and can be written in terms of the partition coefficient. The extraction yield in the top phase of the system can be calculated

with equation 6 in which *R* is the ratio of the volume of the top phase to the volume of the bottom phase:

$$Y_T = \frac{V_T C_T}{V_{total} C_{total}}$$

$$Y_T = \frac{V_T C_T}{V_T C_T + V_B C_B}$$

$$Y_T = \frac{1}{1+\frac{1}{KR}} \qquad [6]$$

The extraction yield may be influenced by volume exclusion and the salting out effect. A high concentration or molecular weight of the polymer phase will result in the volume exclusion effect, while salting out will occur with high salt concentrations.

The extraction of hydrophobic molecules in ATPS occurs due to a difference in the relative hydrophobicity of the two phases. Both components in the ATPS system are hydrophilic with varying relative hydrophobicity. With a PEG-salt ATPS, the PEG solution is more hydrophobic than the salt phase and hydrophobic molecules will selectively dissolve and partition to the PEG phase (top phase). An increase in the PEG concentration may lead to an increase in the partitioning due to the increase in hydrophobic interactions.

It may be possible to achieve effective partitioning of polyphenolic compounds such as resveratrol with tartrate, citrate, carbonate, sulphate or phosphate salts. Phosphates and sulphates may produce waste streams that are more cumbersome to treat. To try and ensure that a digestible product is produced by the methods described herein, edible salts may be used. For instance, citrate salts can form two phases and are biodegradable and non-toxic. The applicant has found that tartrate provides the highest yield of resveratrol from polymer-salt ATPS as compared to phosphate, sulphate and citrate sodium salts.

The salt concentration may influence the partitioning by altering the ionic strength of the system. At a high salt concentration, the ionic strength of the salt phase is high. As will be understood by those skilled in the art, the effect of salt concentration on partitioning depends on the type of molecule to be separated.

The molecular weight of the polymer phase will affect the concentration of polymer required for phase formation. If the polymer molecular weight is high, a lower concentration of the polymer may be used to produce two phases. Conversely, a higher polymer concentration is required if the molecular weight is low. However, a low polymer molecular weight reduces the interfacial tension between the salt and the polymer phases, thereby increasing the partition coefficient. The use of high molecular weight PEG, for example, decreases the partitioning to the top phase, since there is a decrease in the free volume that accompanies the increase in the polymer chain length.

PEG with a molecular weight between 4000 g/mol and 20 000 g/mol is generally used in ATPS for effective partitioning of most biological molecules, including polyphenolic compounds. The applicant has found that a preferred PEG molecular weight for resveratrol extraction with PEG-salt ATPS appears to be about 8000 g/mol.

Another factor influencing the extraction or separation in ATPS is the pH of the system. The pH affects the electrochemical interactions and can be adjusted to improve the separation. Polyphenols such as resveratrol include hydroxyl groups in their chemical structure that dissociate at different rates based on the pH of the surrounding medium. The dissociation constants of resveratrol, for example, are $pK_{a1}$=8.99, $pK_{a2}$=9.63 and $pK_{a3}$=11.4. The pH of the system affects the partitioning of resveratrol because the ionization state of resveratrol shifts as the pH is adjusted. As the pH of the system is increased, the molecule becomes more polar, and it is thus expected that the resveratrol will preferentially partition into the more polar phase of the ATPS.

Further, the temperature affects the binodal curve of an ATPS as well as the viscosity and the density of the components. The temperature does not influence the partition coefficient or yield of a biomolecule. The temperature should, however, be held constant to prevent density and viscosity changes in the two phases.

Protein precipitation may be used as an alternative method to the polymer-polymer ATPS technique to purify and extract polyphenolic compounds from the polymer phase of the polymer-salt ATPS into a protein-polyphenol supplement form, and preferably a supplement with a protein matrix that is rich in resveratrol. Polyphenols such as resveratrol may bind to the protein surface, resulting in complex formation and precipitation. Phenols are capable of precipitating proteins from a solution if they are maintained in solution at concentrations sufficient to push the equilibrium in favour of the protein-phenol complex. This results in the formation of a hydrophobic layer of phenol molecules on the protein surface. Protein precipitations generally occur in a stepwise manner. Sufficient mixing time is required for the protein and the target molecule to collide and for molecules to diffuse. Once the particles reach a critical size, they may continue to grow by colliding and flocculating. The precipitate particles may continue to repeatedly collide, aggregate and then break apart until a stable mean particle size is reached.

Neutral salts, such as ammonium sulphate can be used to aid the precipitation. Most proteins are less soluble in solutions with high salt concentrations due to the additional ions of the salt shielding the protein with multiple ion

charges. The ions compress the solvation layer and increases protein-protein interactions. This results in the proteins aggregating and thereafter precipitating out of the solution.

The recovery of resveratrol, for example, from the extracting phase can be determined by evaluating how much resveratrol had precipitated out of the solution using equation 7 in which $v_o$ and $c_o$ are the volume and concentration of resveratrol in the organic phase after the precipitation and $v_i$ and $c_i$ are the initial volume and initial concentration of resveratrol in solution before precipitation:

$$Extraction\ recovery\ of\ resveratrol\ (\%) = \left[1 - \left(\frac{v_o c_o}{v_i c_i}\right)\right] \times 100\% \qquad [7]$$

## Examples

*Materials*

Grape pomace and grape canes were obtained from the Department of Viticulture and Oenology at the University of Stellenbosch. The pomace was stored for several months below freezing temperature to ensure minimal decay of the fresh product. Fresh canes were pruned from the grape vines and year-old canes were collected from vineyard prunings. A variety of fresh leaves were obtained off the vines, older fallen leaves and leaves suspected to be infected with Roll leaf were also collected. A hammer mill was utilized to reduce the canes to a powdered form at a speed of 1800 rpm. Material was fed into the hammer mill spread evenly over the entire feed at a uniform rate. It was ensured that the material was conveyed continuously at a slow rate to ensure uniform size reduction. The powdered material was discharged via the air chute and collected in a bag.

*Comparative example - Determining the resveratrol content of grape pomace, grape canes and vine leaves*

*Methods*

The resveratrol content of the raw materials was determined after mixing the periods of 30 minutes and over 2 days between the biomass and 80:20 (v/v) ethanol-water solution.

(a) Thirty-minute contacting period

The wet grape pomace was homogenised by blending 40 g of grape pomace with 100 ml of 80:20 (v/v) ethanol-water solution in a bench-top blender (Optima Stainless Steel Blender, Mellerware) for 5 minutes on high power. The homogenised grape pomace was then poured into a graduated reagent bottle and continuously stirred for 30 minutes using a magnetic stirrer with the lid placed on the bottle. The dry grape pomace samples were blended (Optima Stainless Steel Blender, Mellerware) for 5 minutes on high power with 80:20 (v/v) ethanol-water solution. 10.61 g of the dry sample was then poured into a graduated reagent bottle and stirred for 30 minutes with the lid placed on the bottle. The leaves were blended in the benchtop blender for 5 minutes and 5 g of powdered leaf biomass was placed in a graduated reagent bottle. 5 g of the older cane and new cane biomass samples were placed in separate reagent bottles. 50 ml of ethanol-water 80:20 (v/v) solutions were added to each of the three samples, thereafter the mixtures were continuously stirred for 30 minutes using a magnetic stirrer.

Liquid samples were extracted from all the above mixtures and placed in 5 ml centrifuge tubes. The samples were centrifuged for 5 minutes at 14 500 rpm to remove solids and minimize the possibility of stray light influencing the spectrophotometric readings of the samples.

A base solution of 80:20 (v/v) ethanol-water solution was prepared to be used as a baseline for the spectrophotometer. Samples for the absorbance measurement were then prepared by diluting the centrifuged samples with 80:20 (v/v) ethanol-water solution. 0.05 ml of the wet and dry grape pomace solutions were pipetted into in 50 ml of the ethanol-water solutions to provide adequately dilute samples of less than 2.5 abs for analysis. 0.1 ml of the old cane, new cane and leaf samples were diluted in 50 ml of ethanol-water solution.

A Varian Cary 1E UV-Vis spectrophotometer was then used to determine the absorbance of each of the samples. It was ensured that the spectrophotometer had been running for at least an hour before testing commenced. 80:20 (v/v) ethanol-water solution was poured into two quartz cuvettes. These were inserted into the spectrophotometer and used to baseline ('zero') the spectrophotometer. One of the cuvettes was then removed and the solution replaced with the sample to be tested. The cuvette was rinsed using the sample to ensure that there was no residual water in the cuvette which would compromise the accuracy of the measurement. A full scan from 900 nm to 200 nm was then done to determine the peak for resveratrol. The measurement of the absorbance at the peak could then be used to quantify the

resveratrol content in each of the samples. The dilute concentrations of the samples (g/l) were determined using the calibration curve. The actual concentration was calculated by multiplying the dilute concentration by the dilution ratio. The concentration in terms of the grams of dry weight was then determined by multiplying by the volume of the liquid solution and dividing by the mass of the dry mass of the sample.

(b) Contacting over two-day period

The same methods described in section (a) were repeated, except that the samples were mixed continuously over a two-day period using magnetic stirrers to maximise contact between the biomass and the samples.

(c) Wet to dry mass ratio

Frozen grape pomace was dried in the laboratory oven at 60 °C overnight to determine the ratio of wet mass to dry mass of pomace. 160.15 g of wet mass was dried which yielded 42.50 g of dry mass. The ratio of 3.77 wet to dry mass enables the quantification of resveratrol content in terms of grams of dry weight (g d.w.)

*Results*

The resveratrol content of samples of the various types of wine waste, including leaves, dried or wet grape pomace, and either newly cut or year-old canes, were determined after contacting the samples in an 80:20 (v/v) ethanol-water solution for periods of 30 minutes and over 2 days to extract the resveratrol out of the solid biomass. It was observed from the UV-Vis scans of the samples that the amount of resveratrol extracted in each case increased significantly from the 30-minute to 2-day contacting time, indicating that significant time is required to achieve full extraction. It was assumed that after 2 days of contacting, with good mixing, the total extractable resveratrol of the samples had been obtained.

The different concentrations of resveratrol in old grape canes, fresh grape canes, wet grape pomace and dry grape pomace are shown in Figure 4. The wet grape pomace has 14.5 mg resveratrol per gram dry weight and the fresher grape canes has 21 mg resveratrol per gram dry weight. The old canes has a reduced resveratrol content of 14.7 mg resveratrol per gram dry weight and the dried pomace has 9.5 mg resveratrol per gram dry weight. The reduction in resveratrol content in the dried pomace and old canes indicate that resveratrol may be degraded by heat and environmental exposure.

The two stereoisomers of resveratrol absorb at different wavelengths. The *cis-* isomer absorbs between 229-322 nm and the *trans-* isomer between 223-322 nm. The spectra of the *cis-* and the *trans-* isomers differ significantly below wavelengths of 300 nm and are thus distinguishable, but are more difficult to distinguish closer to the 300 nm range. Due to the presence of a single peak of the grape pomace and cane samples falling in close proximity to the 300nm range, it was inferred that the resveratrol contained within the raw biomass samples of the grape canes and grape pomace was predominantly trans-resveratrol. It has also been reported that the trans-isomer is the predominant form found in grapes and vines (Fremont, 2000).

The samples may include the *cis-* form and not the *trans-* form of resveratrol due to isomerization of the *trans-*configuration to the *cis-* configuration after prolonged freezing and storage and subsequent defrosting of the grape pomace used in the experiments. It is recommended that fresh samples of grape pomace and canes be obtained and comparative testing of the samples under different storage conditions be done to validate the influence of external factors on the resveratrol structure. The absorbance peaks observed at absorbances in the range of 200 nm may be indicative of the potential protein content of the samples and were thus not taken into consideration.

The quantification of the dried grape resveratrol content was done to enable comparison of the resveratrol content of the dry versus the wet grape pomace. There was a 55% decrease in the resveratrol content of the in the dry sample, indicating that the process of drying the pomace at elevated temperatures resulted in significant deterioration of resveratrol in the sample. This is a factor that needs to be taken into consideration during the storage and processing of biomass samples to extract resveratrol. The fresher grape canes were found to have 30% more resveratrol than the older grape canes, indicating that the resveratrol content likely degrades with age or exposure to environmental conditions, and fresher canes are preferable for extraction. However, these results should be verified, since the cane samples used were not standardized.

*Example 1 - Polymer-salt ATPS to extract resveratrol with PEG of different molecular weights*

*Methods*

Polymer-salt aqueous two-phase systems with PEG of different molecular weights were prepared using PEG

400 ($M_w$ =400 g/mol), PEG 1000 ($M_w$ =1000 g/mol), PEG 4000 ($M_w$ =4000 g/mol), PEG 8000 ($M_w$ =8000 g/mol), and PEG 10000 ($M_w$ =10 000 g/mol).

Two different ATP systems with different polymer and salt concentrations were investigated. System A contains 11 wt% PEG and 26 wt% ammonium sulphate and system B contains 13 wt% PEG and 13 wt% ammonium sulphate. The quantities of materials used to prepare the ATP systems are summarised in Table 2.

Table 2. Quantities of materials used to extract resveratrol in systems A and B

| | System A | System B |
|---|---|---|
| Volume pomace (ml) | 20 | 20 |
| Volume water (ml) | 20 | 20 |
| Mass PEG (g) | 5 | 5 |
| Mass Ammonium sulphate (g) | 12 | 5 |

The resveratrol partition coefficient and percentage extraction were determined for each system and each different molecular weight of PEG based on the concentration of resveratrol present in grape pomace of 21.31 mg/g dry weight.

*Results*

For the polymer-salt ATPS the target phase where the resveratrol is expected to selectively partition to is the PEG-rich top phase.

The partition coefficient and percentage extraction obtained with both systems A and B at the different PEG molecular weights is given in Tables 3 and 4 and the data is illustrated graphically in Figures 5 and 6.

Table 3. Partition coefficients and percentage extraction of resveratrol achieved with system A

| System A | | |
|---|---|---|
| **PEG** | **Partition Coefficient** | **% Extraction** |
| PEG 400 | 164 | 32 |
| PEG 1000 | 177 | 28 |
| PEG 4000 | 341 | 44 |
| PEG 8000 | 351 | 47 |
| PEG 10000 | 174 | 22 |

Table 4. Partition coefficients and percentage extraction of resveratrol achieved with system B

| System B | | |
|---|---|---|
| **PEG** | **Partition Coefficient** | **% Extraction** |
| PEG 400 | | |
| PEG 1000 | | |
| PEG 4000 | 33 | 27 |
| PEG 8000 | 242 | 58 |
| PEG 10000 | 200 | 56 |

The results may show that the highest partition coefficient and percentage extraction of resveratrol is achieved with PEG with a molecular weight of 8000 g/mol in respect of each of systems A and B.

The results may further show that to obtain significant partitioning of resveratrol into the top polymer phase the ratio of PEG 8000 to salt may range between 1:1 (wt% PEG: wt% salt) and 1:2.5 (wt% PEG: wt% salt).

*Example 2 - The effect of ammonium sulphate salt concentration on the partition coefficient and percentage extraction of resveratrol from grape pomace*

*Methods*

20 ml pomace was added to a polymer-salt ATP systems comprising 20 ml water, 5 g PEG 8000 (11 wt%) and 2.5 g (7 wt%), 5 g (13 wt%), 8 g (19 wt%) and 12 g (26 wt%) ammonium salt, respectively. The partition coefficient and percentage extraction of resveratrol to the PEG top phase was determined for each of the different salt concentrations.

*Results*

The partition coefficients and percentage extraction achieved with polymer-salt ATP systems formed with PEG 8000 and different concentrations of ammonium sulphate is summarised in Table 5 and the results are illustrated graphically in Figures 7 and 8.

Table 5. Partition coefficients and percentage extraction of resveratrol achieved with different ammonium sulphate salt concentrations

| wt% Ammonium sulphate | Partition Coefficient | % Extraction |
|---|---|---|
| 7 | 78 | 58 |
| 13 | 242 | 58 |
| 19 | 334 | 70 |
| 26 | 351 | 46 |

The highest percentage resveratrol was extracted with 19 wt% ammonium sulphate.

*Example 3 - The partition coefficient and percentage extraction of resveratrol from grape pomace with different types* of *salts*

*Methods*

20 ml pomace was added to a polymer-salt ATP systems comprising 11 wt% PEG 8000 and 26 wt% tartrate, citrate, sulphate and phosphate salt solutions (20 ml water), respectively. The partition coefficient and percentage extraction of resveratrol to the PEG top phase was determined for each of the different types of salts.

*Results*

The partition coefficients and percentage extraction achieved with polymer-salt ATP systems formed with 11 wt% PEG 8000 and 26 wt% pf different types of salt are summarised in Table 6 and the results are illustrated graphically in Figures 9 and 10.

Table 6. Partition coefficients and percentage extraction of resveratrol achieved with different types of salts

| Salt Type | wt% Salt | Partition Coefficient | % Extraction |
|---|---|---|---|
| Potassium sodium tartrate tetrahydrate | 25,5 | 177 | 67 |
| Tri-sodium citrate dihydrate | 25,5 | 79 | 32 |
| Ammonium sulphate | 25,5 | 351 | 46 |
| Potassium hydrogen phosphate trihydrate | 25,5 | 218 | 54 |

The highest percentage resveratrol was extracted with potassium sodium tartrate tethrahydrate.

*Example 4 - Determination of an effective polymer-salt ATPS for the highest yield of resveratrol from biomass*

Further experiments demonstrated that an effective polymer-salt combination for the extraction of resveratrol in terms of achievable yields is a system comprising about 10% (w/w) PEG 8000 and about 15% (w/w) tartrate salt. The highest total yield that was achieved in extracting resveratrol from the raw biomass was 70%, with 99.6% of the resveratrol partitioning to the top phase. The bottom phase of the ATPS (consisting of the salt solution) therefore only contained trace amounts of resveratrol, with the remaining resveratrol still contained in the biomass. It was therefore assumed that within the bottom phases exiting the polymer-salt ATPS reactor, that the remaining resveratrol that was not extracted would be contained within the raw biomass of grape pomace and canes, and not in the salt solution.

*Example 5 - Protein precipitation of resveratrol at different protein solution concentrations of tryptone soy broth and yeast extract*

*Methods*

Yeast extract and tryptone soy broth powder are sources of protein for protein-polyphenol precipitation. Firstly, solutions of 0.05 g/ml, 0.1 g/ml, 0.15 g/ml, 0.2 g/ml and 0.3 g/ml protein were prepared by adding demineralized water to 0.5 g, 1 g, 1.5 g, 2 g and 3 g or protein powder to a total volume of 10 ml.

A PEG-resveratrol solution was prepared by mixing 12.5 g of PEG 8000, 0.1 g of resveratrol and 50 ml of demineralized water. A blank solution containing no resveratrol was prepared to be used to baseline the spectrophotometer by mixing 12.5 g of PEG and 50 ml of demineralized water. 0.1 ml of the PEG-resveratrol and 0.1 ml of the PEG-blank solution were diluted to 20 ml with water. The spectrophotometer was zeroed using two quartz cuvettes of the diluted blank solution. A scan was done on the diluted PEG-resveratrol solution to determine the initial resveratrol concentration in the solution.

5 ml of the protein solutions were added to 5 ml of the PEG-resveratrol solutions and 5 ml of the protein solutions were added to 5 ml solutions of the PEG-blank solutions. The samples were then stirred using a magnetic stirrer for 30 minutes to allow for sufficient mixing of the protein and the polyphenol. Thereafter the samples were pipetted into 4 ml centrifuge tubes and centrifuged at 14 000 rpm for 5 minutes to spin the protein precipitate out of the solution. The remaining solution after centrifuging was analysed with a spectrophotometer to determine the remaining resveratrol concentration in the PEG solution. The process was repeated with the addition of 0.1 g, 0.2 g and 0.3 g of NaCl to determine the impact of salt addition and the potential salting out effect associated with precipitation.

*Results*

Protein precipitations with tryptone soy broth and yeast extract as sources of protein were investigated to recover the resveratrol from the PEG-rich top-phase of the polymer-salt ATPS into a protein/resveratrol supplement form.

As the protein concentration of the samples increases, the resveratrol concentration of the samples may steadily decrease, corresponding to the precipitation of the compound as protein/resveratrol complexes. For example, the absorbance peak of samples containing the same volume and resveratrol content decreased from 2.59 abs to 0.15 abs as the protein concentration of the system was increased from 0.025 g/ml to 0.15 g/ml.

It was observed that in a protein/PEG system of 0.075 g/ml yeast extract concentration, no resveratrol was present in the solution. The UV-Vis scan of the solution had no absorbance peak in the resveratrol wavelength range. This indicates that most or all of the resveratrol had precipitated out of the solution and a recovery of almost 100% of resveratrol into the precipitate was achieved with the yeast extract solution with a concentration of 0.15 g/ml. To scale up the process, various other, preferably less expensive, protein powders may be used for the recovery of resveratrol.

The results of the triplicate tests performed for each protein solution concentration are shown in Figure 11. An average recovery of about 99.94% was observed for tryptone soy broth systems of 0.15 g/ml and recoveries of almost 100% were achieved for yeast extract protein systems of 0.075 g/ml.

Soy protein powder may be selected for large-scale production of resveratrol/protein powder supplements. Based on the results of the tests, systems with protein concentrations of 0.15 kg soy protein per liter should be sufficient to extract most, if not all, of the resveratrol from the PEG-resveratrol solution

*Example 6 - Resveratrol recovery yields in a 1:1 (wt%:wt%) PEG 8000 and maltodextrin A TPS with different protein solutions*

*Methods*

Polymer-polymer ATP systems were prepared comprising 33 wt% PEG 8000; 33 wt% maltodextrin; 1 g/l resveratrol and 10 g/l of one of the group consisting of albumin, yeast extract, and tryptone soy broth. The percentage resveratrol recovered from the 1 g/l solution of resveratrol by protein precipitation was determined.

*Results*

The percentage resveratrol recovered from a 1 g/l solution in a 33 wt% PEG 8000 system with 10 g/l of albumin, yeast extract or tryptone soy broth solution and a 33 wt% maltodextrin solution is shown in Figure 12. The highest percentage of resveratrol was recovered with the yeast extract.

*Experiment 7 - Polymer-polymer ATPS system with PEG of different molecular weights*

*Methods*

Polymer-polymer aqueous two-phase systems were prepared using maltodextrin (mdx) in combination with PEG 4000 and PEG 8000. A system comprising about 10% (w/w) PEG 8000 and about 35% (w/w) mdx was prepared by adding 30 g of PEG 8000, 70 g of mdx and 0.05 g of resveratrol to a reagent bottle. Demineralised water was added to yield a net weight of 200 g of solution. A system of 10% (w/w) PEG 4000 and 40% (w/w) mdx was also formed by adding 80 g mdx, 20 g PEG 4000, 0.05 g of resveratrol and demineralised water to achieve a total solution weight of 200 g.
Control/base solutions were prepared to be used as a baseline in the spectrophotometer by preparing the same solutions described above, but without resveratrol.
The aqueous two-phase systems were formed by continuously stirring the solutions using a magnetic stirrer for 30 minutes. The pH of the systems was adjusted to selected values below, between and above each of the three $pK_a$ values of resveratrol by adding sodium hydroxide or hydrochloric acid dropwise as required. The solution with the resveratrol to be extracted and the blank solutions were poured into 50 ml graduated centrifuge tubes and centrifuged for 20 minutes at 4400 rpm. In both the extraction solution and in the base solution two distinct phases were formed. 1 ml of the bottom phase of the extraction solution was separated from the system using a pipette tapped into an Erlenmeyer flask and 1 ml of the bottom phase of the blank solution was poured into a separate Erlenmeyer flask. The solutions were diluted by adding 50 ml of demineralised water to each.
Cuvettes containing the base solution were placed in the spectrophotometer and the system baselined. One of the cuvettes was then removed, rinsed with the diluted top phase of the extraction solution, and filled with the solution. A full scan from 900-200nm was done to measure the resveratrol peak. The absorbance at the peak was then used to quantify the resveratrol content.
The resveratrol content of the top phase was quantified using the same methodology as for the bottom phase, using the top phase of the base solution to baseline the spectrophotometer. Triplicate runs were carried out to ensure repeatability and accuracy.

*Results*

For the polymer-polymer ATPS using PEG and mdx, the target phase where the resveratrol is expected to selectively partition to is the bottom mdx-rich phase.
The molecular weight of the polymer has an effect on the partitioning of resveratrol in polymer-polymer ATPS. It is expected that a higher molecular weight polymer would be the most suitable to utilize in combination with mdx to extract resveratrol due to the hydrophobicity of resveratrol.

(a) *Two-phase formation*

Cloud point testing was done to verify the two phase formation of the PEG/mdx polymer-polymer ATPS. ATPS systems of about 10% (w/w) PEG 8000 and about 35% (w/w) mdx, and about 10% (w/w) PEG 4000 and about 40% (w/w) mdx were prepared. Both systems appeared cloudy after their formation, but after mixing for 30 minutes and centrifuging for 30 minutes, the expected 50:50 volume separation occurred.

(b) *The effect of pH adjustment*

A PEG-resveratrol sample using *trans*-resveratrol powder and PEG 8000 was made to replicate the top phase extracted from the PEG/tartrate ATPS that would theoretically enter the polymer-polymer ATPS for further purification. Mdx and water were added to yield the appropriate weight compositions of each of the systems.
The ionisation state of resveratrol changes upon the adjustment of the pH, which in turn affects the partitioning of resveratrol in the ATPS system. The pH of each system was adjusted to four different values: below, between and

above each $pK_a$ value of resveratrol to determine the effect of the pH on the yield of resveratrol into the mdx phase. The first sample represents a pH below the lowest $pK_a$ of 8.99, the second sample lies between the $pK_a$ values of 8.99 and 9.63, the third lies between the $pK_a$ values of 9.63 and 10.64 and the fourth lies above the highest $pK_a$ of 10.64.

The UV-Vis scans had two distinct peaks for the resveratrol, one at a range of 215-225 nm and a second at 305-340 nm. This split peak may indicate isomerisation of the resveratrol. High pH values catalyse isomerisation between the *trans*- and *cis*- forms of resveratrol. This was taken into account in the recovery calculation. The first peak corresponds to the *cis*-isomeric form of resveratrol and the second peak at the higher wavelength the *trans*-isomeric form. The total resveratrol content was quantified using the appropriate *trans*- and *cis*- resveratrol calibration curves.

At a pH of 9.85, it appeared that there is no resveratrol in the bottom phase of the PEG 4000/mdx ATPS as no resveratrol peak was observed in the UV-Vis scan. Consequently, it was assumed that all of the resveratrol had partitioned into the top phase. At a pH of 10.75 and greater than pH 11, the formation of two phases were not observed in the PEG 4000/mdx system.

The PEG 8000/mdx system had yields of resveratrol in the bottom mdx phase that were greater than that of the PEG 4000/mdx at each pH value investigated as shown in Figure 13. It therefore appears that the higher molecular weight PEG had a stronger partitioning effect on the resveratrol. The PEG 8000/mdx ATPS is more suitable for the extraction of resveratrol from the PEG-rich phase to the mdx-rich phase than the PEG 4000/mdx ATPS.

The concentration of both the *trans*- and *cis*- forms of resveratrol in the top (PEG rich) and bottom (mdx rich) phases of the PEG 8000/mdx system were compared and the results are shown in Figures 14 and 15. It can be seen that as the pH of the ATPS increases the concentration of resveratrol in the top phase decreases while the concentration of resveratrol in the bottom phase increases. More resveratrol partitions to the bottom mdx-rich phase as the pH of the system is increased. The highest yield of 58% of separated resveratrol was obtained at a pH of 11.03, which is above the highest $pK_a$ value if resveratrol in the PEG 8000 10% (w/w) / mdx 35% (w/w) ATPS system. The partition coefficient of the system was calculated to be 1.36, verifying that a successful resveratrol recovery into the mdx-rich phase has been achieved.

Mdx is more polar than PEG due to the many hydroxyl groups present in the structure of mdx. When the pH of a solution of resveratrol is increased, resveratrol itself becomes more polar and thus preferentially partitions into the mdx-rich bottom phase.

Since the PEG 8000/mdx system provides a yield of 58% of resveratrol separated into the bottom mdx-rich phase, the system may be considered for scale-up of the polymer-polymer ATPS to extract resveratrol into the bottom mdx-rich phase from the PEG-rich top phase of the PEG/tartrate ATPS.

It has been demonstrated herein that the polyphenolic compound, resveratrol, can be extracted and separated from biomass by polymer-salt ATPS. The polymer-salt ATPS separation may be followed by a polymer-polymer ATPS separation step to achieve a yield of approximately 58% resveratrol. It has also been shown that protein precipitation can be used to separate and recover resveratrol from a polymer phase and that recovery of almost 100% resveratrol in a protein precipitate is possible at suitably high protein concentrations.

ATPS is considered an integrated process as it combines several functions or tasks such as concentration, biomass removal and product purification in one operation. ATPS is able to separate and recover polyphenolic compounds such as resveratrol with relatively few processing steps.

ATPS may have advantages including low energy consumption and low capital costs. It may be capable of continuous operation, may have fast mass transfer and may be relatively easy to scale-up. The chemicals used are environmentally benign and non-toxic, inexpensive and recyclable. The waste from the process requires little or no treatment before disposal. ATPS may therefore be relatively inexpensive and safe. The use of ATP technology may increase processing speed, reduce instrumental complexity and provide scalability as a result of the use of simple machinery such as disc stack centrifuges and settling tanks. Column contactors in which phase separation occurs by gravity are commonly used for organic aqueous phase extractions and can be adopted in the case of ATPS to avoid the use of expensive centrifuges in large scale production.

A polymer-salt ATPS may have lower operating cost than a polymer-polymer ATPS and the lower viscosity between the two phases may make unit operation easier.

ATPS is an environmentally friendly and economical method of extracting polyphenolic compounds such as resveratrol. A problem encountered with the use of solvents to extract resveratrol, for example, is the subsequent recovery and concentration of the resveratrol from the solvent phase into a supplement form. A possible advantage of using one ATPS and protein precipitation or two ATP systems in series to extract the resveratrol into a polymer phase that is safe to consume, is that it may be possible to process the resveratrol into a supplement together with the consumable protein or polymer.

Trans-resveratrol is an easily oxidized, reactive and photosensitive compound, like many other natural polyphenolic compounds. It is difficult to maintain the purity and integrity of resveratrol and other polyphenols during processing. Resveratrol, in particular, oxidises when exposed to air. Furthermore, resveratrol is a hydrophobic compound and has a low aqueous solubility of 3 mg/100 ml. Resveratrol thus does not easily dissolve in water, which generally necessitates

extraction and processing of the compound with solvents which may lead to further degradation thereof. ATP systems generally provide a gentle environment for phase separation while other liquid-liquid extraction (LLE) methods may damage biological target compounds, such as resveratrol, due to their harsher processing conditions and the use of organic solvents. ATPS generally does not degrade biomolecules and provides high separation yields, high capacity and the possibility of polymer and salt recycling. Furthermore, ATP systems have the ability to handle soluble and insoluble matter, there may be low interfacial tension of phases and short durations required for phase separation, and the systems may be capable of delivering a highly-purified product. Mdx/PEG ATPS systems, in particular, may be non-toxic, non-flammable and biodegradable.

Furthermore, the methods described herein provide a way to valorise winery waste.

Throughout the specification, unless the context requires otherwise:

- the word 'comprise' or variations such as 'comprises' or 'comprising' will be understood to imply the inclusion of a stated integer or group of integers but not the exclusion of any other integer or group of integers; and
- w/w ratios are calculated with reference to water weight, i.e. water present in the final composition of reference.

## References

Aggarwal, B.B., Bhardwaj, A., Aggarwal, R.S., Seeram, N.P., Shishodia, S. & Takada, Y. 2004. Role of resveratrol in prevention and therapy of cancer: Preclinical and clinical studies. Anticancer Research. 24(5 A):2783-2840.

Bishayee, A. 2009. Cancer Prevention and Treatment with Resveratrol: From Rodent Studies to Clinical Trials. Cancer Prevention Research. 2(5):409-418.

Bishayee, A., Darvesh, A.S., Politis, T. & McGory, R. 2010. Resveratrol and liver disease: from bench to bedside and community. Liver International. 30(8):1103-1114.

Bosutti, A. & Degens, H. 2015. The impact of resveratrol and hydrogen peroxide on muscle cell plasticity shows a dose-dependent interaction. Scientific reports. 5:8093.

Burns, J., Yokota, T., Ashihara, H., Lean, M.E.J. & Crozier, A. 2002. Plant Foods and Herbal Sources of Resveratrol. J. Agric. Food Chem. 3337-33340.

Fremont, L. 2000. Biological effects of resveratrol. Life sciences. 66(8):663-73.

Matos, M. 2013. Production of emulsions with controlled droplet size containing bioactive compounds using membranes. 1-111.

Morris, V.L., Toseef, T., Nazumudeen, F.B., Rivoira, C., Spatafora, C., Tringali, C. & Rotenberg, S.A. 2015. Antitumor properties of cis-resveratrol methylated analogs in metastatic mouse melanoma cells. Molecular and Cellular Biochemistry. 402(1-2):83-91.

Orallo, F. 2006. Comparative studies of the antioxidant effects of cis- and trans-resveratrol. Current medicinal chemistry. 13(1):87-98.

Rayne, S., Karacabey, E. & Mazza, G. 2008. Grape cane waste as a source of trans-resveratrol and transviniferin: High-value phytochemicals with medicinal and anti-phytopathogenic applications. Industrial Crops and Products. 27(3):335-340.

Romero-Perez, A.I., Lamuela-Raventos, R.M., Andres-Lacueva, C. & De La Carmen Torre-Boronat, M. 2001. Method for the quantitative extraction of resveratrol and piceid isomers in grape berry skins. Effect of powdery mildew on the stilbene content. Journal of Agricultural and Food Chemistry. 49(1):210-215.

Rossi, D., Guerrini, A., Bruni, R., Brognara, E., Borgatti, M., Gambari, R., Maietti, S. & Sacchetti, G. 2012. Trans-resveratrol in nutraceuticals: Issues in retail quality and effectiveness. Molecules. 17(10):12393-12405.

## Claims

1. A method for separating a polyphenolic compound from biomass, the method comprising feeding the biomass into an aqueous two-phase system with a polymer phase and a salt phase and separating the polymer phase which includes the polyphenolic compound from the salt phase, **characterized in that** the polyphenolic compound is resveratrol and **in that** the salt is selected from the group consisting of a tartrate salt, a citrate salt, a carbonate salt, a phosphate salt, a sulphate salt, and any combinations thereof.

2. The method as claimed in Claim 1, wherein the polymer is a polyether selected from the group consisting of polyethylene glycol and polypropylene glycol.

3. The method as claimed in any one of the preceding claims, wherein the biomass is selected from the group consisting of grape pomace, grape canes, vine leaves, peanuts, cocoa powder, blueberries, raspberries, mulberries, cranberries, bilberries, geraniums of the *Pelargonium* genus, the herb *Polygonum Cuspidatum,* and any combinations thereof.

4. The method as claimed in any one of the preceding claims, wherein the polymer phase comprises from 5% to 15% (w/w) polyethylene glycol with a molecular weight in a range from 2,000 g/mol to 10,000 g/mol, and the salt phase comprises from 10% to 20% (w/w) tartrate salt.

5. The method as claimed in any one of Claims 1 to 4, which includes recovering the polyphenolic compound at least partially from the polymer phase by performing protein precipitation to yield a polyphenol-protein precipitate.

6. The method as claimed in any one of Claims 1 to 5, which includes recovering the polyphenolic compound at least partially from the polymer phase by feeding the polymer phase into a second aqueous two-phase system with a second polymer phase and separating the second polymer phase which includes the polyphenolic compound from the polymer phase fed into the second aqueous two-phase system.

7. The method as claimed in any one of the preceding claims, which includes at least one recycling step selected from the group consisting of:

   recycling the polymer phase after at least partially recovering the polyphenolic compound therefrom, and reusing the polymer phase in the aqueous two-phase system to separate a polyphenolic compound from the biomass; and
   recycling the salt phase of the aqueous two-phase system by separating the biomass from the salt phase and reusing the salt phase in the aqueous two-phase system to separate a polyphenolic compound from the biomass.

## Patentansprüche

1. Verfahren zum Abtrennen einer polyphenolischen Verbindung aus Biomasse, wobei das Verfahren das Einspeisen der Biomasse in ein wässriges Zweiphasensystem mit einer Polymerphase und einer Salzphase und das Abtrennen der Polymerphase, die die polyphenolische Verbindung enthält, von der Salzphase umfasst, **dadurch gekennzeichnet, dass** die polyphenolische Verbindung Resveratrol ist und dass das Salz ausgewählt ist aus der Gruppe bestehend aus einem Tartratsalz, einem Citratsalz, einem Carbonatsalz, einem Phosphatsalz, einem Sulfatsalz und beliebigen Kombinationen davon.

2. Verfahren wie in Anspruch 1 beansprucht, wobei das Polymer ein Polyether ist, ausgewählt aus der Gruppe bestehend aus Polyethylenglykol und Polypropylenglykol.

3. Verfahren wie in einem der vorhergehenden Ansprüche beansprucht, wobei die Biomasse ausgewählt ist aus der Gruppe bestehend aus Traubentrester, Weinstöcken, Weinblättern, Erdnüssen, Kakaopulver, Blaubeeren, Himbeeren, Maulbeeren, Preiselbeeren, Heidelbeeren, Geranien aus der Gattung *Pelargonium,* dem Kraut *Polygonum Cuspidatum* und beliebigen Kombinationen davon.

4. Verfahren wie in einem der vorhergehenden Ansprüche beansprucht, wobei die Polymerphase 5% bis 15% (w/w) Polyethylenglykol mit einem Molekulargewicht in einem Bereich von 2.000 g/mol bis 10.000 g/mol umfasst und die Salzphase 10% bis 20% (w/w) Tartratsalz umfasst.

5. Verfahren wie in einem der Ansprüche 1 bis 4 beansprucht, welches eine Rückgewinnung der polyphenolischen Verbindung zumindest teilweise aus der Polymerphase durch Durchführen einer Proteinfällung, um ein Polyphenol-Protein-Präzipitat zu erhalten, einschließt.

6. Verfahren wie in einem der Ansprüche 1 bis 5 beansprucht, welches eine Rückgewinnung der polyphenolischen Verbindung zumindest teilweise aus der Polymerphase durch Einspeisen der Polymerphase in ein zweites wässriges Zweiphasensystem mit einer zweiten Polymerphase und Trennen der zweiten Polymerphase, die die polyphenolische Verbindung umfasst, von der Polymerphase, die in das zweite wässrige Zweiphasensystem eingespeist wurde, einschließt.

7. Verfahren wie in einem der vorhergehenden Ansprüche beansprucht, welches mindestens einen Recyclingschritt umfasst, der ausgewählt ist aus der Gruppe bestehend aus:

Recyceln der Polymerphase nach zumindest teilweiser Rückgewinnung der polyphenolischen Verbindung daraus und Wiederverwenden der Polymerphase in dem wässrigen Zweiphasensystem, um eine polyphenolische Verbindung von der Biomasse abzutrennen; und
Recyceln der Salzphase des wässrigen Zweiphasensystems durch Abtrennen der Biomasse von der Salzphase und Wiederverwenden der Salzphase in dem wässrigen Zweiphasensystem, um eine polyphenolische Verbindung von der Biomasse abzutrennen.

## Revendications

1. Procédé pour séparer un composé polyphénolique d'une biomasse, le procédé comprenant l'introduction de la biomasse dans un système aqueux à deux phases ayant une phase de polymère et une phase saline et la séparation de la phase de polymère qui inclut le composé polyphénolique de la phase saline, **caractérisé en ce que** le composé polyphénolique est le resvératrol et **en ce que** le sel est choisi dans le groupe constitué par un sel de tartrate, un sel de citrate, un sel de carbonate, un sel de phosphate, un sel de sulfate et toutes associations de ceux-ci.

2. Procédé selon la revendication 1, dans lequel le polymère est un polyéther choisi dans le groupe constitué par le polyéthylène glycol et le polypropylène glycol.

3. Procédé selon l'une quelconque des revendications précédentes, dans lequel la biomasse est choisie dans le groupe constitué par le marc de raisin, les rafles de raisin, les feuilles de vigne, les cacahuètes, la poudre de cacao, les myrtilles, les framboises, les mûres blanches, les airelles, les myrtilles des marais, les géraniums du genre *Pelargonium,* l'herbe *Polygonum Cuspidatum,* et toutes associations de ceux-ci.

4. Procédé selon l'une quelconque des revendications précédentes, dans lequel la phase de polymère comprend de 5 % à 15 % (p/p) de polyéthylène glycol ayant un poids moléculaire dans une plage allant de 2 000 g/mol à 10 000 g/mol, et la phase saline comprend de 10 % à 20 % (p/p) de sel de tartrate.

5. Procédé selon l'une quelconque des revendications 1 à 4, qui inclut la récupération du composé polyphénolique au moins partiellement à partir de la phase de polymère en effectuant une précipitation des protéines pour donner un précipité de polyphénol-protéine.

6. Procédé selon l'une quelconque des revendications 1 à 5, qui inclut la récupération du composé polyphénolique au moins partiellement à partir de la phase de polymère en introduisant la phase de polymère dans un deuxième système aqueux à deux phases ayant une deuxième phase de polymère et en séparant la deuxième phase de polymère qui inclut le composé polyphénolique de la phase de polymère introduite dans le deuxième système aqueux à deux phases.

7. Procédé selon l'une quelconque des revendications précédentes, qui inclut au moins une étape de recyclage choisie dans le groupe constitué par :

le recyclage de la phase de polymère après en avoir récupéré au moins partiellement le composé polyphénolique, et en réutilisant la phase de polymère dans le système aqueux à deux phases pour séparer un composé polyphénolique de la biomasse ; et
le recyclage de la phase saline du système aqueux à deux phases en séparant la biomasse de la phase saline

et en réutilisant la phase saline dans le système aqueux à deux phases pour séparer un composé polyphénolique de la biomasse.

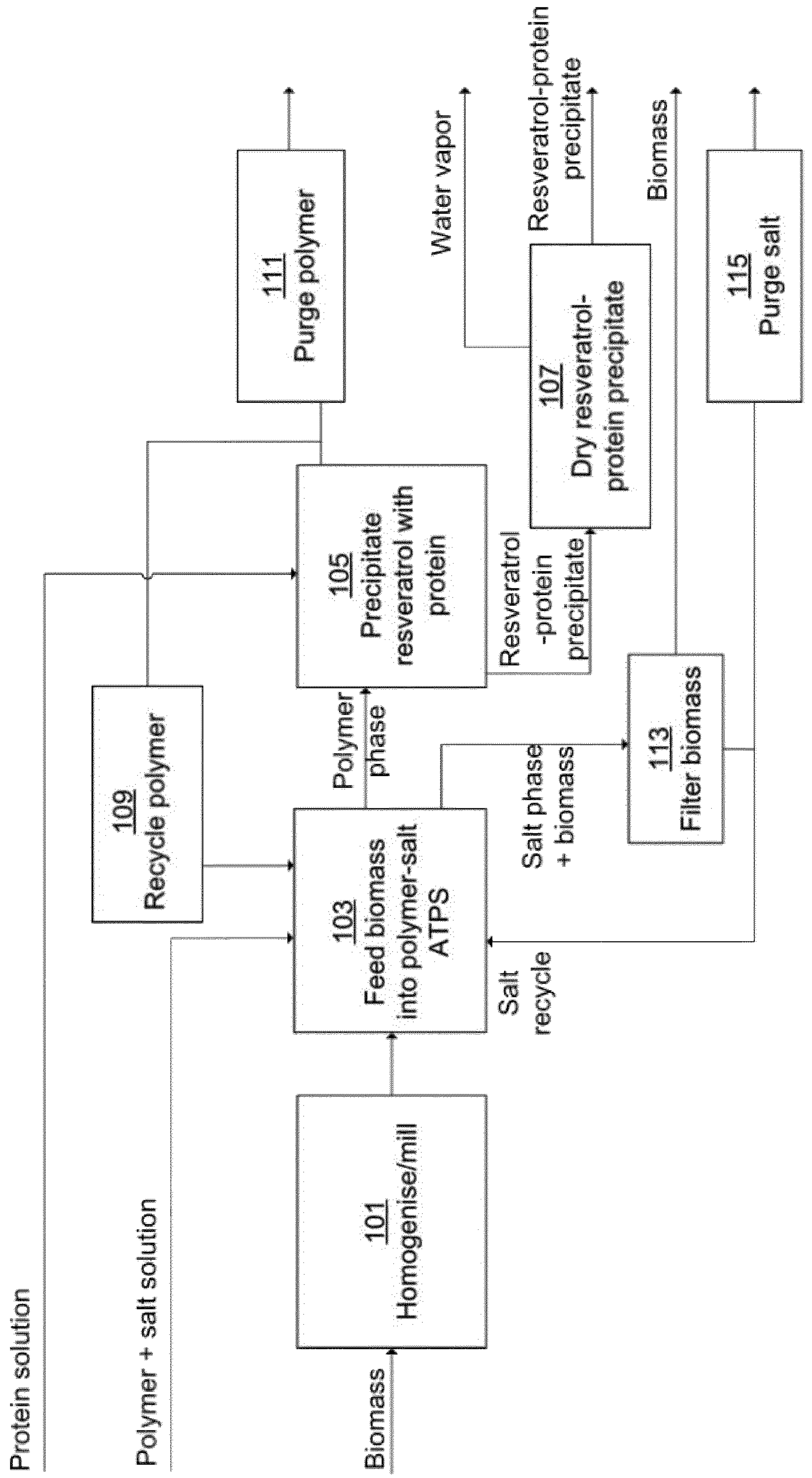
Protein solution
Polymer + salt solution
Biomass
101
Homogenise/mill
103
Feed biomass
into polymer-salt
ATPS
109
Recycle polymer
Polymer
phase
Salt phase
+ biomass
Salt
recycle
105
Precipitate
resveratrol with
protein
Resveratrol
-protein
precipitate
113
Filter biomass
111
Purge polymer
107
Dry resveratrol-
protein precipitate
Water vapor
Resveratrol-protein
precipitate
Biomass
115
Purge salt

FIGURE 1

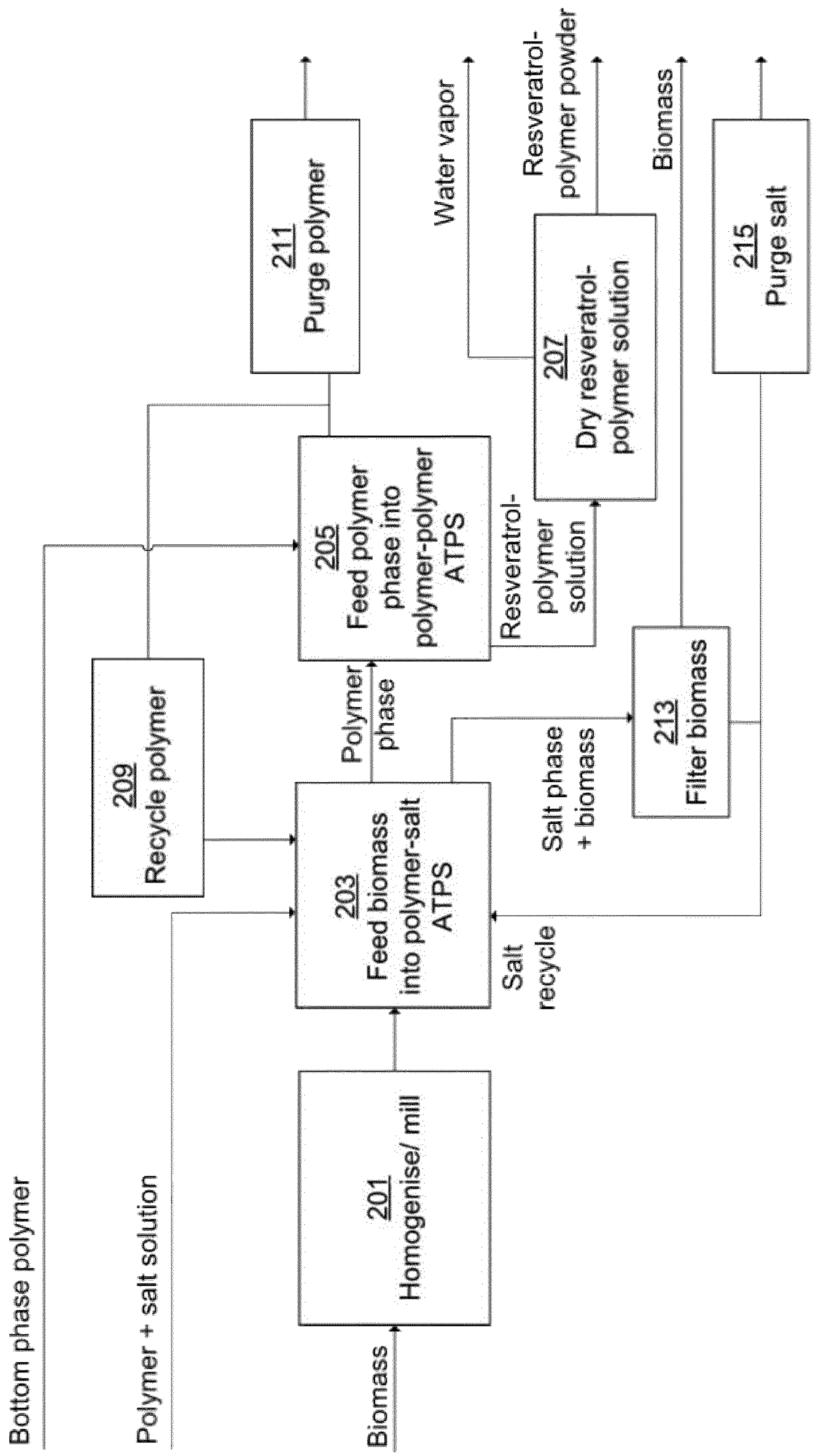
Bottom phase polymer
Polymer + salt solution
Biomass
201
Homogenise/ mill
203
Feed biomass into polymer-salt ATPS
Salt recycle
209
Recycle polymer
Polymer phase
205
Feed polymer phase into polymer-polymer ATPS
Resveratrol-polymer solution
Salt phase + biomass
213
Filter biomass
211
Purge polymer
207
Dry resveratrol-polymer solution
Water vapor
Resveratrol-polymer powder
Biomass
215
Purge salt

FIGURE 2

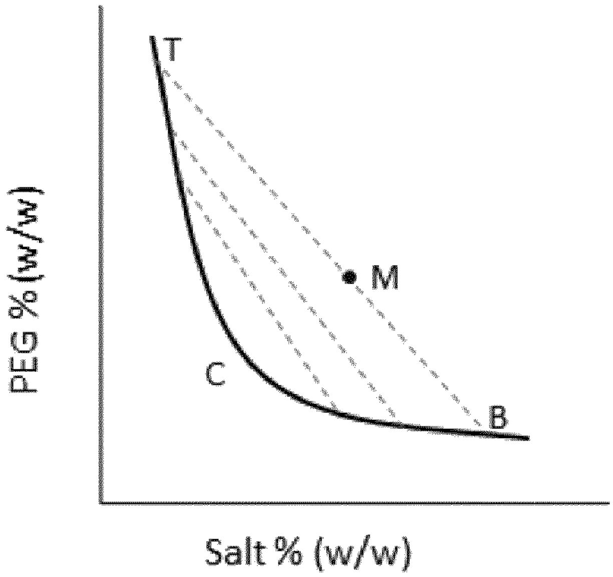


FIGURE 3

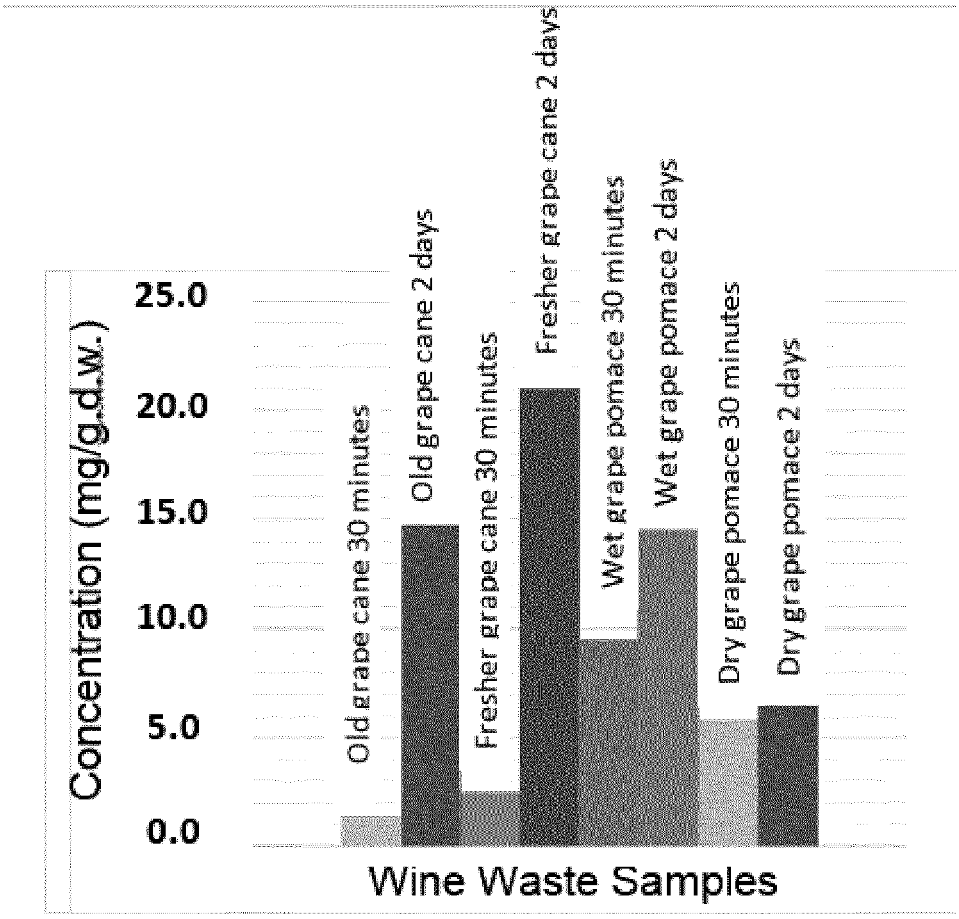


FIGURE 4

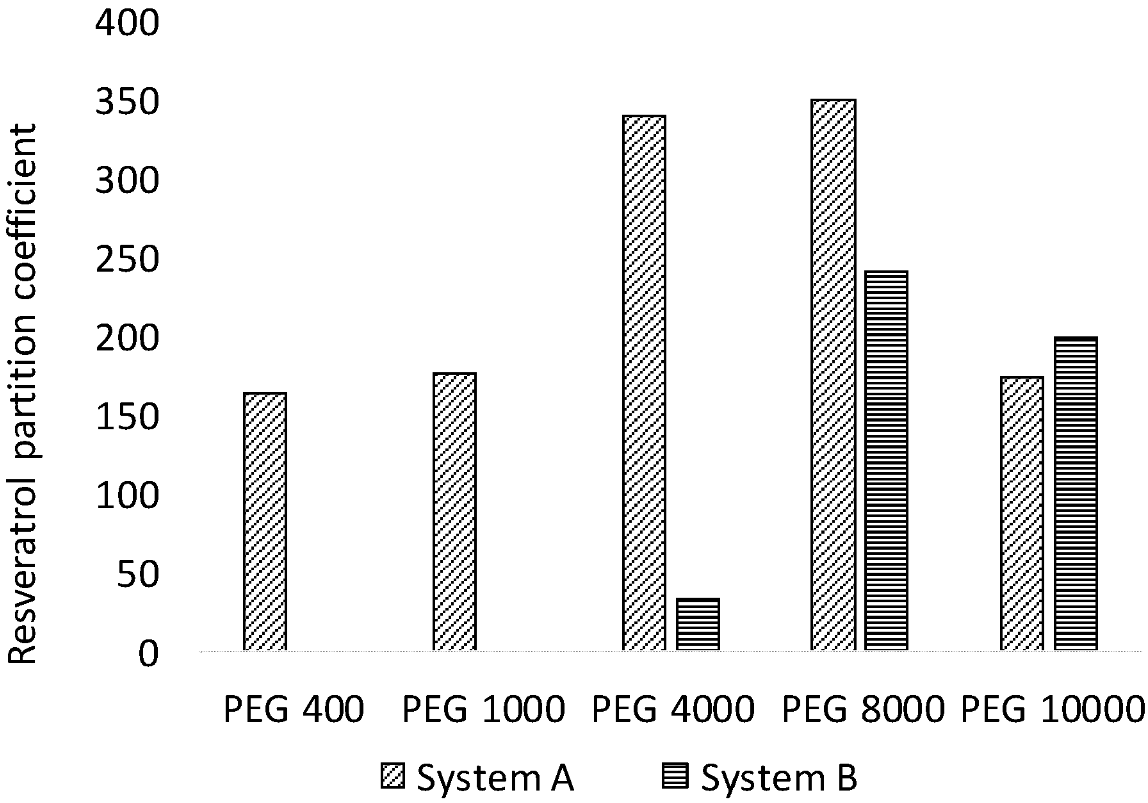


FIGURE 5

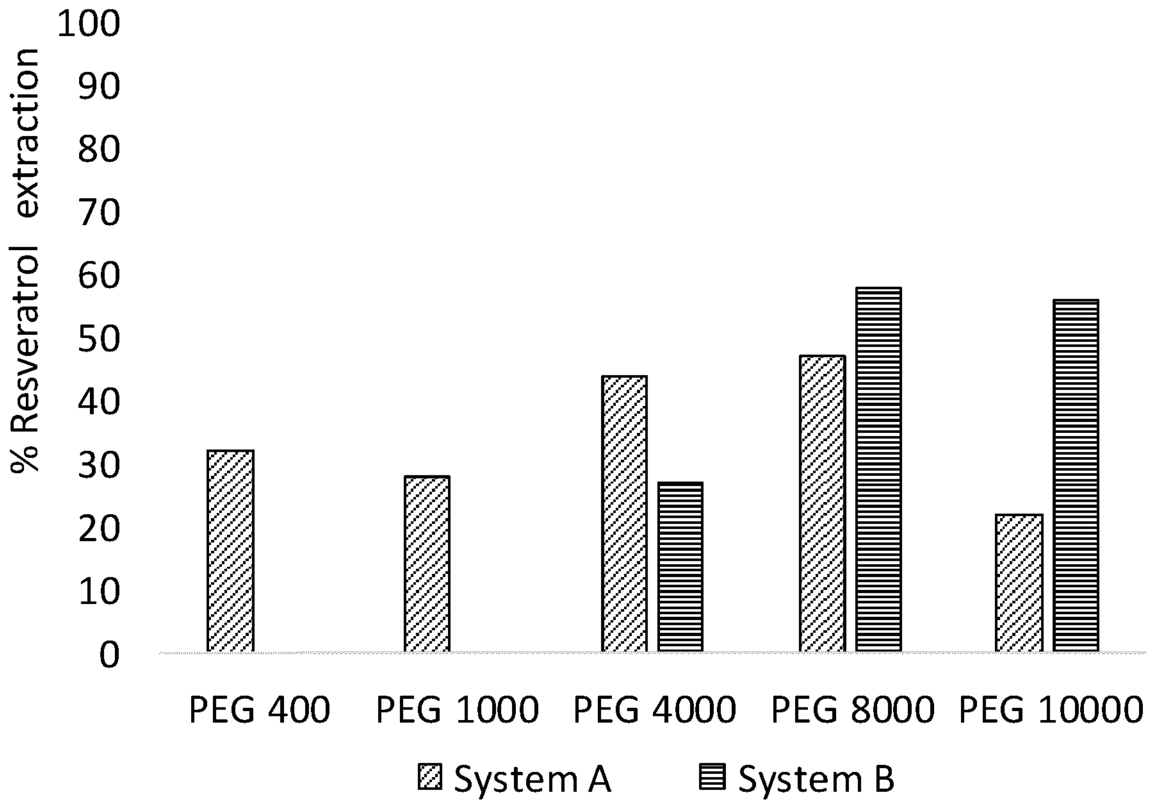


FIGURE 6

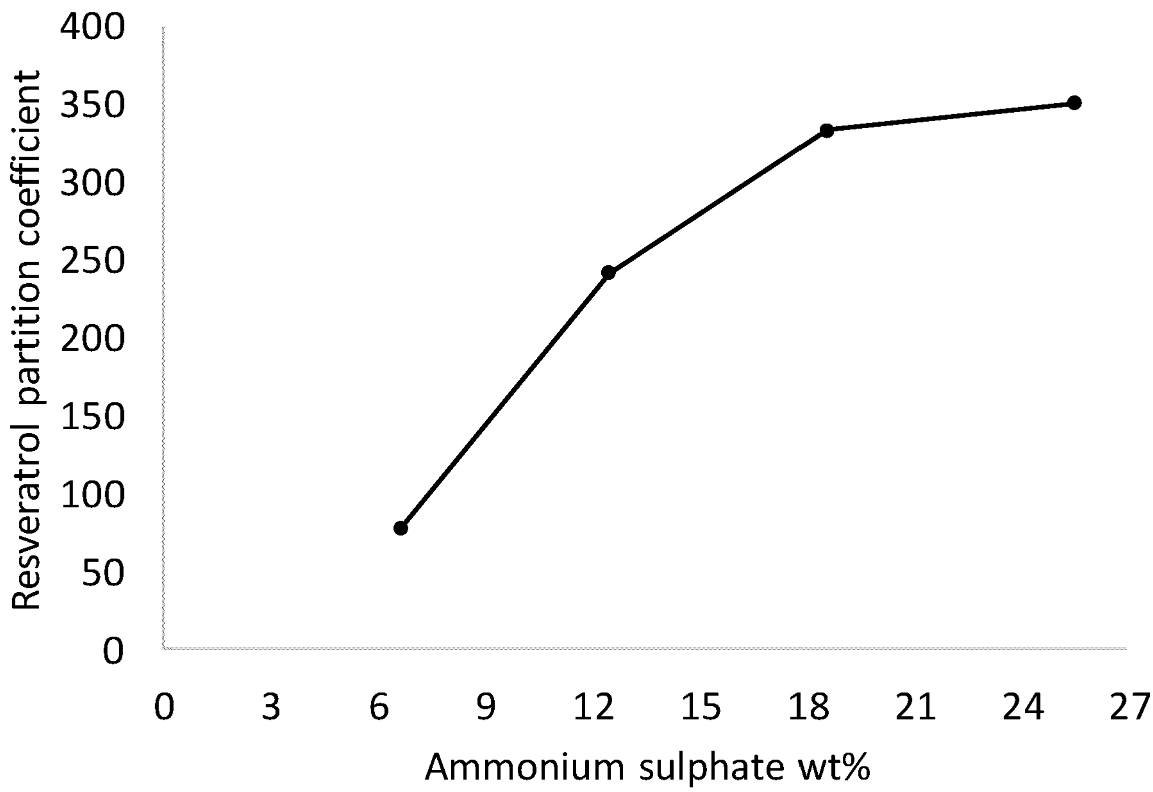


FIGURE 7

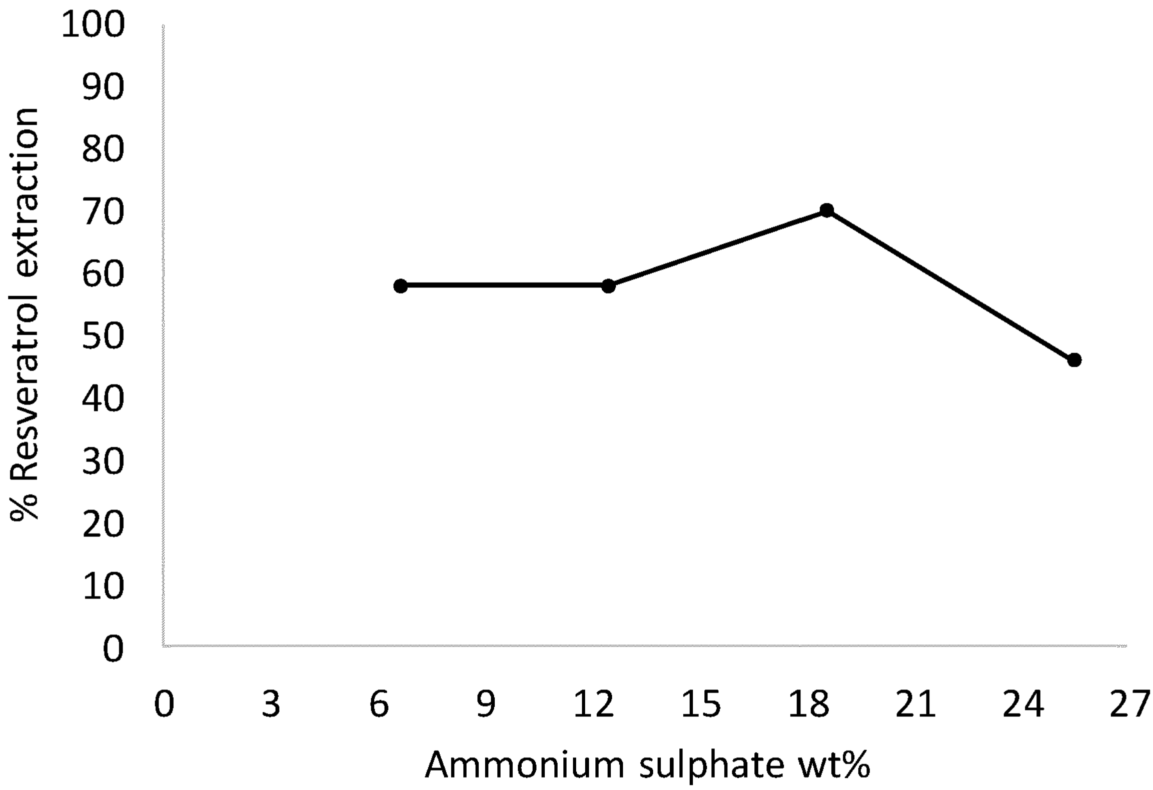


FIGURE 8

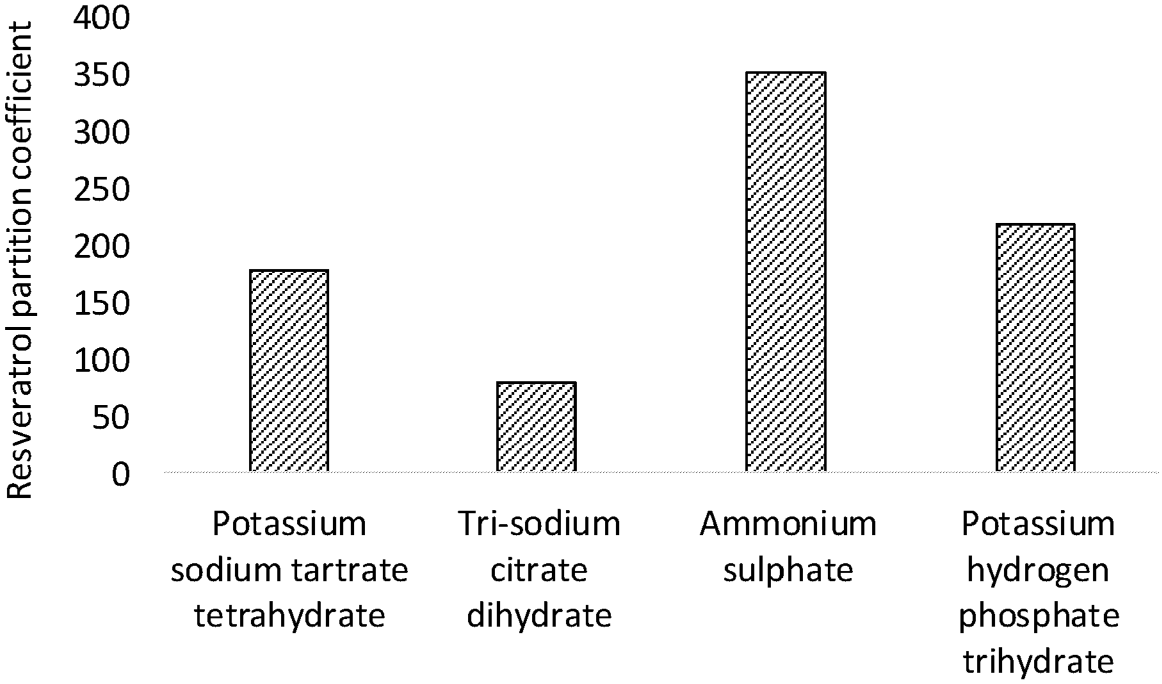


FIGURE 9

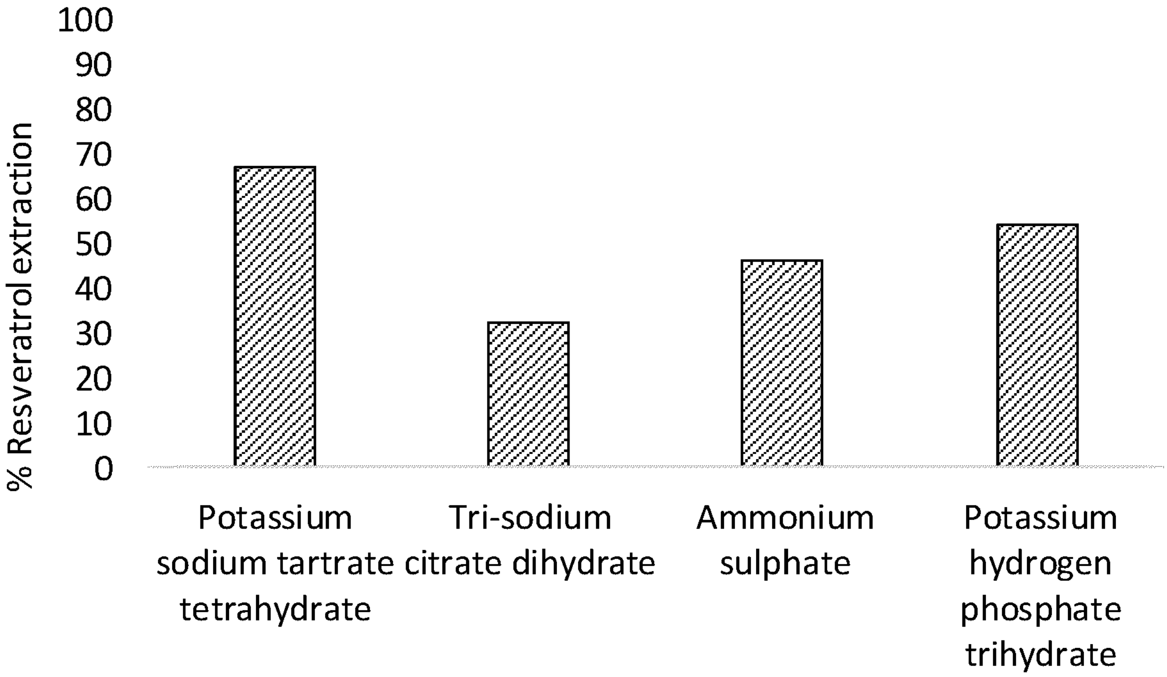


FIGURE 10

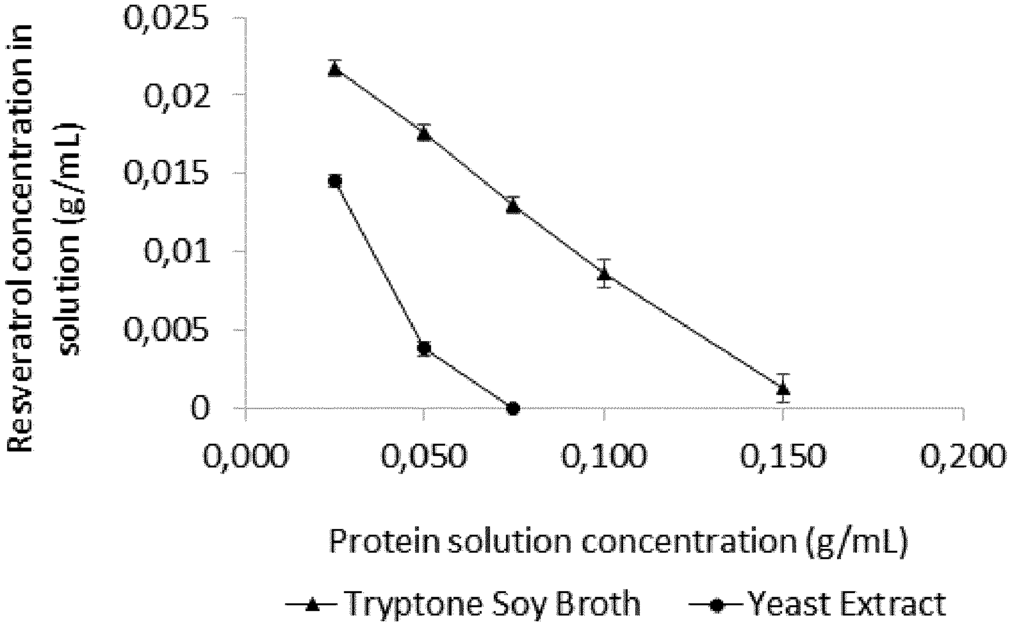


FIGURE 11

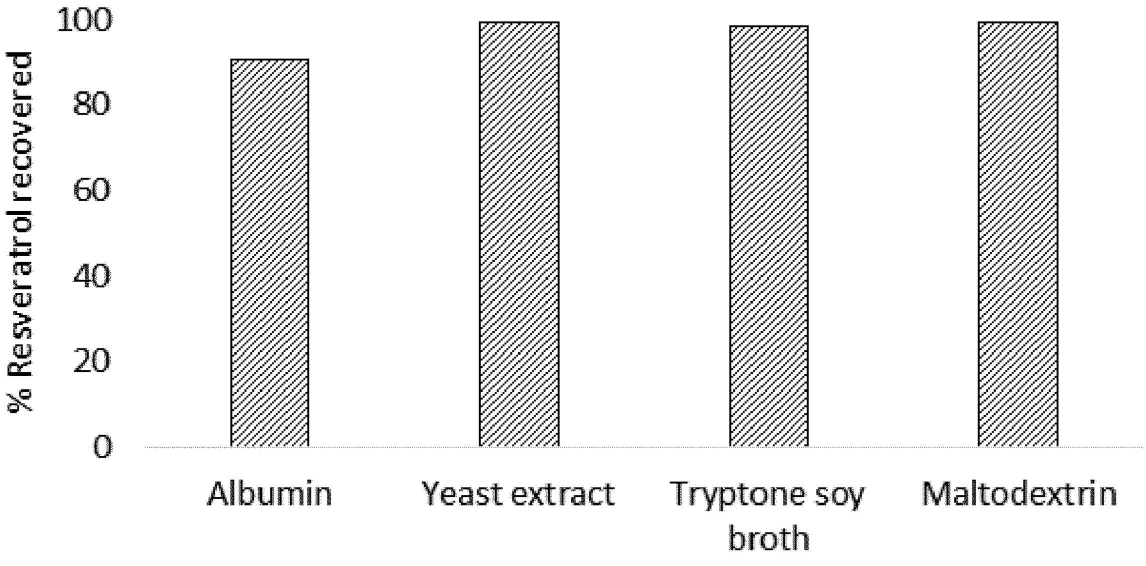


FIGURE 12

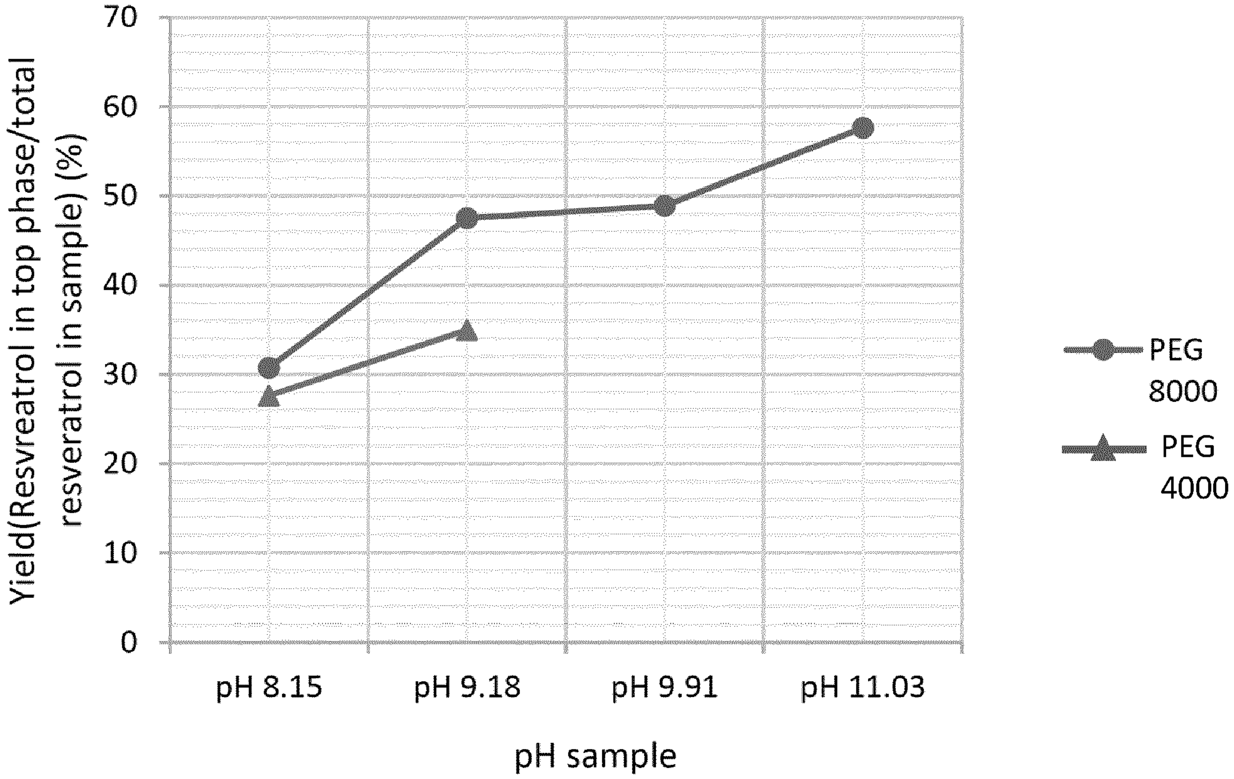


FIGURE 13

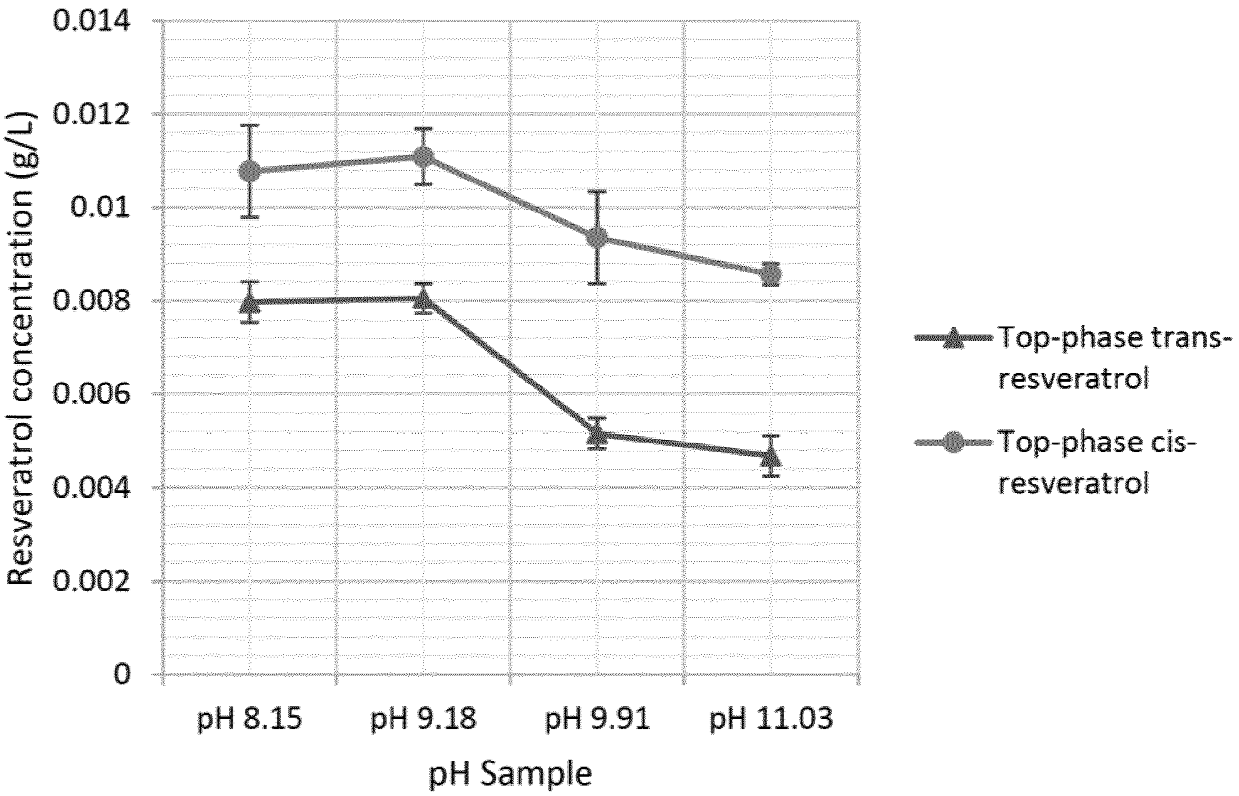


FIGURE 14

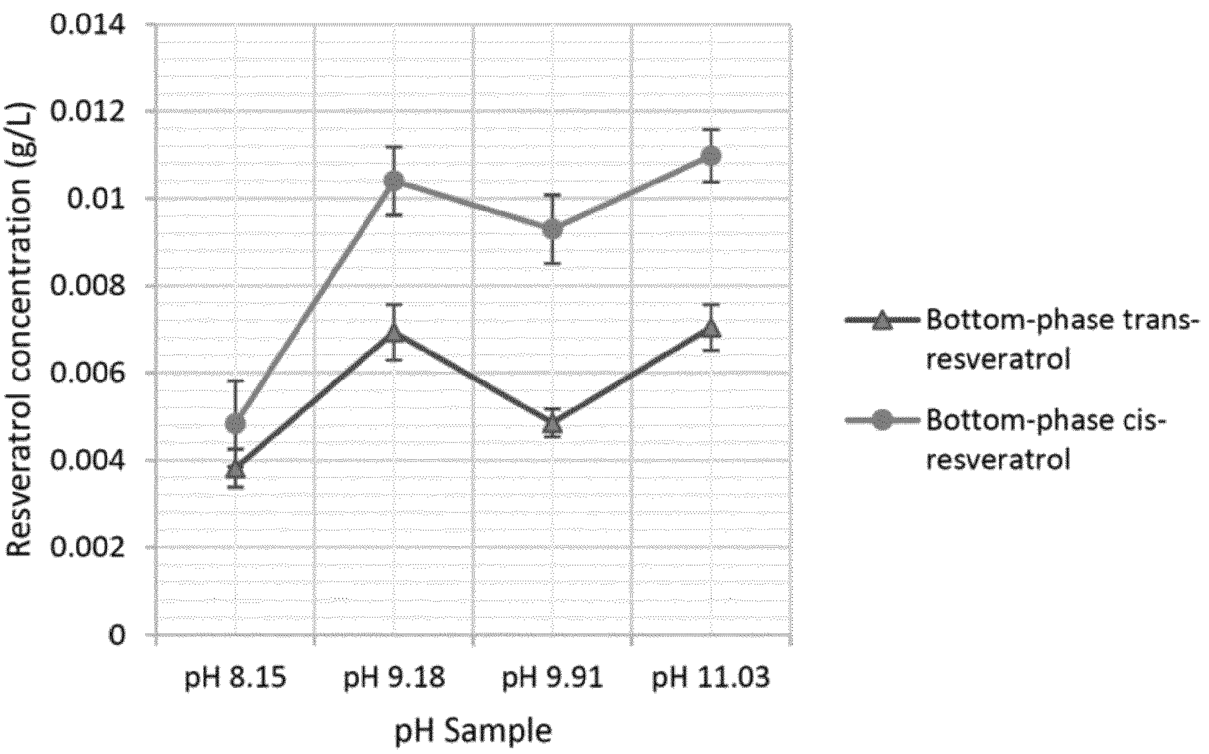
0.014
0.012
0.01
0.008
0.006
0.004
0.002
0
Resveratrol concentration (g/L)
pH 8.15
pH 9.18
pH 9.91
pH 11.03
pH Sample
Bottom-phase trans-resveratrol
Bottom-phase cis-resveratrol

FIGURE 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107840839 A


### Non-patent literature cited in the description


- **LUCIA XAVIER et al.** *Maderas. Ciencia Y Technologia,* 2015, 0-0
- **SAILI J. IDURKAR et al.** *Separation Science and Technology,* 2018, vol. 54 (1), 51-58
- **XAVIER LUCIA et al.** *Waste and Biomass Valorization,* 2016, vol. 8 (2), 443-452
- **MARIO A. TORRES-ACOSTA et al.** *Biotechnology Journal,* 2019, vol. 14 (1), 1800117
- **JACQUELINE HERBST et al.** *Journal of Chemical Engineering,* 2019, vol. 64 (7
- **HUI WANG et al.** *Biotechnology letters,* 2008, vol. 30 (12), 2079-2084
- **AGGARWAL, B.B. ; BHARDWAJ, A. ; AGGARWAL, R.S. ; SEERAM, N.P. ; SHISHODIA, S. ; TAKADA, Y.** Role of resveratrol in prevention and therapy of cancer: Preclinical and clinical studies. *Anticancer Research,* 2004, vol. 24 (5 A), 2783-2840
- **BISHAYEE, A.** Cancer Prevention and Treatment with Resveratrol: From Rodent Studies to Clinical Trials. *Cancer Prevention Research,* 2009, vol. 2 (5), 409-418
- **BISHAYEE, A. ; DARVESH, A.S. ; POLITIS, T. ; MCGORY, R.** Resveratrol and liver disease: from bench to bedside and community. *Liver International,* 2010, vol. 30 (8), 1103-1114
- **BOSUTTI, A. ; DEGENS, H.** The impact of resveratrol and hydrogen peroxide on muscle cell plasticity shows a dose-dependent interaction. *Scientific reports,* 2015, vol. 5, 8093
- **BURNS, J. ; YOKOTA, T. ; ASHIHARA, H. ; LEAN, M.E.J. ; CROZIER, A.** Plant Foods and Herbal Sources of Resveratrol. *J. Agric. Food Chem.,* 2002, 3337-33340
- **FREMONT, L.** Biological effects of resveratrol. *Life sciences,* 2000, vol. 66 (8), 663-73
- **MATOS, M.** *Production of emulsions with controlled droplet size containing bioactive compounds using membranes,* 2013, 1-111
- **MORRIS, V.L. ; TOSEEF, T. ; NAZUMUDEEN, F.B. ; RIVOIRA, C. ; SPATAFORA, C. ; TRINGALI, C. ; ROTENBERG, S.A.** Anti-tumor properties of cis-resveratrol methylated analogs in metastatic mouse melanoma cells. *Molecular and Cellular Biochemistry,* 2015, vol. 402 (1-2), 83-91
- **ORALLO, F.** Comparative studies of the antioxidant effects of cis- and trans-resveratrol. *Current medicinal chemistry,* 2006, vol. 13 (1), 87-98
- **RAYNE, S. ; KARACABEY, E. ; MAZZA, G.** Grape cane waste as a source of trans-resveratrol and transviniferin: High-value phytochemicals with medicinal and anti-phytopathogenic applications. *Industrial Crops and Products,* 2008, vol. 27 (3), 335-340
- **ROMERO-PEREZ, A.I. ; LAMUELA-RAVENTOS, R.M. ; ANDRES-LACUEVA, C. ; DE LA CARMEN TORRE-BORONAT, M.** Method for the quantitative extraction of resveratrol and piceid isomers in grape berry skins. Effect of powdery mildew on the stilbene content. *Journal of Agricultural and Food Chemistry,* 2001, vol. 49 (1), 210-215
- **ROSSI, D. ; GUERRINI, A. ; BRUNI, R. ; BROGNARA, E. ; BORGATTI, M. ; GAMBARI, R. ; MAIETTI, S. ; SACCHETTI, G.** Trans-resveratrol in nutraceuticals: Issues in retail quality and effectiveness. *Molecules,* 2012, vol. 17 (10), 12393-12405